# EUROPEAN PATENT APPLICATION

(11) **EP 4 410 785 A1**
(43) Date of publication of application: **07.08.2024**
(21) Application number: 22872177.5
(22) Date of filing: 26.09.2022
(51) Int. Cl.: C07D 401/12, C07D 239/545, C07D 403/12, C07D 417/12, C07D 487/04, C07D 413/12, A61P 11/00, A61P 11/06, A61P 11/14, A61P 29/00

(54) **DIHYDROPYRIMIDINE COMPOUND, PREPARATION METHOD THEREFOR AND USE THEREOF**

(30) Priority: 27.09.2021 CN 202111137108
(71) Applicant: Chengdu Shibeikang Biomedical Technology Co., Ltd., Chengdu, Sichuan 611730 (CN)
(72) Inventor: ZENG, Yanqun, Chengdu, Sichuan 611730 (CN); HUANG, Long, Chengdu, Sichuan 611730 (CN); ZHU, Xucheng, Chengdu, Sichuan 611730 (CN); ZHU, Tao, Chengdu, Sichuan 611730 (CN); MOU, Xia, Chengdu, Sichuan 611730 (CN); FU, Haixia, Chengdu, Sichuan 611730 (CN)
(74) Representative: Herrero & Asociados, S.L.
(86) International application number: PCT/CN2022/121194
(87) International publication number: WO 2023/046133

(57) **Abstract**

Disclosed are a dihydropyrimidine based compound, a preparation method therefor and an application thereof, belonging to the technical field of pharmaceutical chemistry. The present invention relates to a dihydropyrimidine based compound, the structure of which is shown in formula I. Also provided in the present invention is an application of the compound represented by formula I, or a salt, solvate, isomer, metabolite, nitrogen oxide and prodrug thereof, in the preparation of a medicament for treating or preventing P2X3 and/or P2X2/3 receptor-related diseases. The dihydropyrimidine based compound of the present invention has good P2X3 receptor antagonist activity and improved safety.

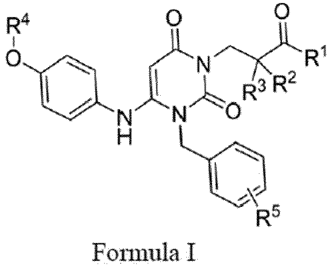

## Description

This application claims priority to a Chinese patent application No. 202111137108.1 filed to the Chinese Patent Office on September 27, 2021 under the title Dihydropyrimidine based Compound, Preparation Method and Use Thereof, the entire contents of which are incorporated herein by reference.

### Technical Field

The present invention relates to the technical field of pharmaceutical chemistry, and particularly to a dihydropyrimidine-based compound and the preparation method and use of the same.

### Background

It is estimated that 5-10% of adults worldwide suffer from chronic cough. Some of these patients who have refractory chronic cough (RCC) and unexplained chronic cough (UCC) are more sensitive to a variety of triggers that do not normally cause coughing in healthy subjects, including daily activities (e.g., talking and laughing), changes in temperature, exposure to aerosols, or food volatile. To date, treatment options for these patients have been extremely limited, and many have often gone years without relief. There are no approved medications for chronic cough. Commonly used clinical medications such as codeine and dextromethorphan, which are central cough suppressants, frequently lead to adverse effects such as constipation and drowsiness. Statistics shows that chronic cough with a duration of >8 weeks accounts for more than 1/3 of respiratory outpatient cases, with a prevalence of 2-10%.

P2X3 receptors, a member of the purine receptor family, are non-selective ligand-gated ion channels that play an important role in the generation and transmission of injurious information. Recent studies have found that overactivation of P2X3 receptors is associated with hyper-sensitization of sensory neurons. Neuronal hypersensitivity in the airways and lungs induced by injury or infection can lead to excessive, persistent and frequent coughing. P2X3 receptors have been implicated in a variety of disorders, including chronic cough, neuralgia, and sleep apnea, all of which have high prevalence rates and large unmet clinical needs. A New Drug Application (NDA) has been filed with the FDA for Merck & Co's new cough suppressant gefapixant (MK-7264), an oral, selective P2X3 receptor antagonist for the treatment of adult patients with refractory chronic cough (RCC) or unexplained chronic cough (UCC). Two phase III clinical trials have demonstrated a statistically significant reduction in 24-hour cough frequency (the number of coughs per hour measured objectively using 24-hour recording) at Week 12 (COUGH-1 study) and Week 24 (COUGH-2 study) in the treatment group with a 45 mg dose of gefapixant twice a day compared to the placebo group. In these two studies, the primary efficacy endpoint was not met in the treatment group with a 15 mg dose of gefapixant twice a day. The clinical endpoint was met in the 45 mg group, but there was a higher frequency of discontinuation due to adverse events and a higher incidence of taste-related adverse events with 45 mg. Therefore, there is a clinical need for a safer and more effective P2X3 receptor antagonist.

### Summary of the Invention

One object of the present invention is to provide a dihydropyrimidine-based compound, which has improved P2X3 receptor antagonism and improved safety.

It is another object of the present invention to provide a method for preparing the compound.

It is yet another object of the present invention to provide the use of the compound.

In order to achieve the above objects, the technical solutions employed in the present invention are as follows.

The present invention provides a compound having a structure represented by Formula I, or a salt, a solvate, an isomer, a metabolite, a nitrogen oxide, or a prodrug thereof: 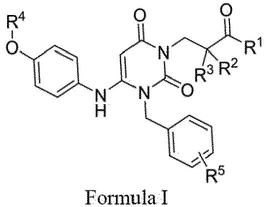
wherein R¹ is selected from hydroxyl, a substituted or unsubstituted alkoxy, a substituted or unsubstituted sulfonamide group, a substituted or unsubstituted heterocyclic epoxy group, and a substituted or unsubstituted aryloxy;
R² and R³ are independently selected from hydrogen, deuterium, and a substituted or unsubstituted C₁₋₁₂ alkyl; or R² and R³ connect to each other to form a 3 to 15-membered cycloalkyl;
R⁴ is selected from a substituted or unsubstituted heterocyclic group, and a substituted or unsubstituted aryl;
R⁵ is selected from hydrogen, deuterium, a substituted or unsubstituted alkyl, a substituted or unsubstituted alkoxy, a substituted or unsubstituted alkylthio, a substituted or unsubstituted amino, and halogen;
and the compound of formula I does not include the compounds shown in Table 1:

**Table 1**

| | | | |
|---|---|---|---|
| A | 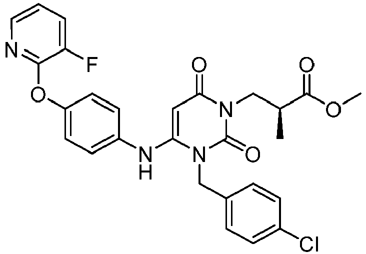 | B | 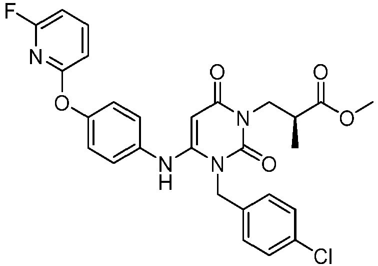 |
| C | 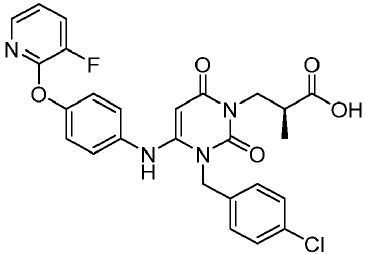 | D | 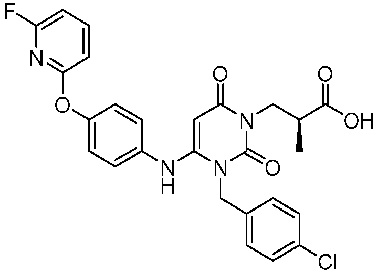 |
| E | 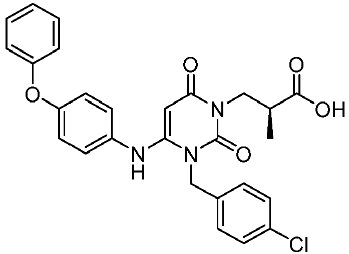 | \ | \ |

In some embodiments of the present invention, R⁴ is selected from substituted or unsubstituted phenyl, substituted or unsubstituted pyridyl, substituted or unsubstituted pyrimidinyl, substituted or unsubstituted pyrazinyl, substituted or unsubstituted pyridazinyl, substituted or unsubstituted benzothiazolyl, substituted or unsubstituted benzisoxazolyl, substituted or unsubstituted imidazopyridazinyl.

In some embodiments of the present invention, R¹ is selected from hydroxyl, substituted or unsubstituted alkoxy, substituted or unsubstituted sulfonamide group;
and/or R⁴ is selected from a substituted or unsubstituted phenyl, a substituted or unsubstituted pyridyl, a substituted or unsubstituted pyrimidinyl, a substituted or unsubstituted pyrazinyl, a substituted or unsubstituted pyridazinyl, a substituted or unsubstituted benzothiazolyl, a substituted or unsubstituted benzisoxazolyl, and a substituted or unsubstituted imidazopyridazinyl, wherein the substituent of R⁴ is selected from 1 to 5 of hydrogen, deuterium, amino, cyano, halogen, a substituted or unsubstituted alkyl, and a substituted or unsubstituted alkoxy;
and/or **R⁵** is selected from 1 to 5 hydrogen, deuterium, amino, cyano, halogen, and a substituted or unsubstituted alkyl.

In some embodiments of the present invention, R² and R³ are independently selected from hydrogen, deuterium, substituted or unsubstituted methyl, substituted or unsubstituted ethyl, substituted or unsubstituted propyl, and substituted or unsubstituted cyclopropyl;
preferably, when R² and R³ are independently selected from substituted methyl, substituted ethyl, substituted propyl, or substituted cyclopropyl, the substituent of R² and R³ are independently selected from one or more deuterium, halogen, hydroxyl, amino, methyl, ethyl, cyclopropyl, tert-butyl, methoxy, ethoxy, cyclopropoxy, tert-butoxy, methylthio, ethylthio, cyclopropylthio, tert-butylthio, amino, methylamino, ethylamino, cyclopropylamino, tert-butylamino, formamido, acetamido, cyclopropylamido, tert-butylamido, NO₂, CN, and CF₃;
or R² and R³ connect to each other to form substituted or unsubstituted cyclopropyl, cyclohexyl, or cyclopentyl; wherein the substituent of the substituted cyclopropyl is selected from one or more deuterium, halogen, hydroxyl, amino, methyl, ethyl, cyclopropyl, tert-butyl, methoxy, ethoxy, cyclopropoxy, tert-butoxy, methylthio, ethylthio, cyclopropylthio, tert-butylthio, amino, methylamino, ethylamino, cyclopropylamino, tert-butylamino, formamido, acetamido, cyclopropylamido, tert-butylamido, NO₂, CN, and CF₃; and/or R¹ is selected from hydroxyl, substituted or unsubstituted alkoxy, substituted or unsubstituted sulfonamide group;
preferably, R¹ is selected from a substituted or unsubstituted alkoxy, and a substituted or unsubstituted sulfonamide group, and the substituent of R¹ is selected from one or more deuterium, halogen, hydroxyl, alkyl, cycloalkyl, alkenyl, alkynyl, aryl, alkoxy, cycloalkoxy, alkylamino, cycloalkylamino, alkylthio, cycloalkylthio, amido, NO₂, CN, and CF₃;
and/or R⁴ is selected from a substituted or unsubstituted phenyl, a substituted or unsubstituted pyridyl, a substituted or unsubstituted pyrimidinyl, a substituted or unsubstituted pyrazinyl, a substituted or unsubstituted pyridazinyl, a substituted or unsubstituted benzothiazolyl, a substituted or unsubstituted benzisoxazolyl, and a substituted or unsubstituted imidazopyridazinyl; the substituent of R⁴ is selected from 1 to 5 deuterium, amino, cyano, methyl, methoxy, halogen, trifluoromethoxy, and difluoromethoxy;
and/or R⁵ is selected from 1 to 5 hydrogen, deuterium, amino, halogen, trifluoromethyl, and difluoromethyl.

### In some embodiments of the present invention,

R² and R³ are independently selected from hydrogen, deuterium, methyl, ethyl, propyl, and cyclopropyl;
or R² and R³ connect to each other to form cyclopropyl;
and/or R¹ is selected from hydroxyl, a C₁-C₁₆ alkoxy, a C₁-C₁₆ alkylsulfonamide group, a C₁-C₆ alkylaminosulfonamide group, and a C₁-C₆ cycloalkylaminosulfonamide group, and a substituted or unsubstituted benzenesulfonamide group;
and/or R⁴ is selected from substituted or unsubstituted phenyl, substituted or unsubstituted pyridyl, substituted or unsubstituted pyrimidinyl, substituted or unsubstituted pyrazinyl, substituted or unsubstituted pyridazinyl, substituted or unsubstituted benzothiazolyl, substituted or unsubstituted benzisoxazolyl, substituted or unsubstituted imidazopyridazinyl, wherein the substituent is selected from 1 to 5 deuterium, amino, cyano, methyl, methoxy, halogen, trifluoromethoxy, and difluoromethoxy;
and/or R⁵ is selected from 1-5 hydrogen, deuterium, amino, methyl, halogen, trifluoromethyl, and difluoromethyl.

In some embodiments of the present invention, R¹ is selected from hydroxyl, a C₁-C₁₆ alkoxy, a C₁-C₁₆ alkylsulfonamide group, a C₁-C₆ alkylaminosulfonamide group, and a C₁-C₆ cycloalkylaminosulfonamide group.

In some embodiments of the present invention, R⁴ is selected from 4-phenyl, 2-pyridinyl, 2-pyrimidinyl, 2-pyrazinyl, 3-pyridazinyl, 3-fluoropyridin-2-yl, (5-trifluoromethoxy)pyridin-2-yl, (5-difluoromethoxy)pyridin-2-yl, 5-chloro-pyridin-2-yl, 3-pyridinyl, 4-pyridinyl, 6-fluoro-pyridin-2-yl, 2-cyanopyrimidin-5-yl, 3-methylpyrazin-2-yl, 2-chloro-pyridin-4-yl, 6-methoxypyrimidin-3-yl, 5-chloropyridin-3-yl, 2-methoxypyrimidin-4-yl, 2-cyanopyridin-4-yl, 3-chloropyrazin-2-yl, 6-chloropyrimidin-4-yl, 5-methylpyridin-2-yl, 3-chloropyridin-2-yl, 5-methoxypyridin-2-yl, 5-chloropyridin-2-yl, benzo[D]thiazol-2-yl, imidazo[1,2-B]pyridazin-6-yl, benzo[D]isoxazol-3-yl, 3,6-difluoropyridin-2-yl, and 3-chloro-6-fluoropyridin-2-yl.

In some embodiments of the present invention, the compound is selected from the compounds shown in Table 2 below or a pharmaceutically acceptable salt thereof.

**Table 2**

| | | |
|---|---|---|
| 1 | 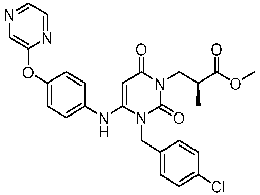 | methyl S-3-(3-(4-chlorobenzyl)-2,6-dioxo-4-(4-(pyrazin-2-oxyloxyl)phenyl)amino)-3,6-dihydropyrimidin-1(2H)-yl)-2-methylpropionate |
| 2 | 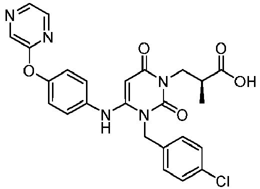 | (S)-3-(3-(4-chlorobenzyl)-2,6-dioxo-4-(4-(pyrazin-2-oxyl)phenyl)amino)-3,6-dihydropyrimidin-1(2H)-yl)-2-methylpropionic acid |
| 3 | 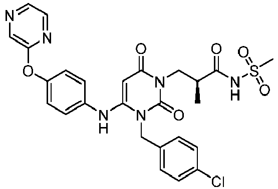 | (S)-3-(3-(4-chlorobenzyl)-2,6-dioxo-4-(4-(pyrazin-2-oxyl)phenyl)amino)-3,6-dihydropyrimidin-1(2H)-yl)-2-methyl-N-(methylsulfonyl)propionamide group |
| 4 | 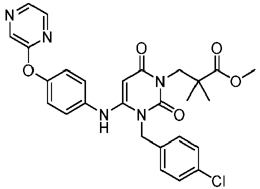 | Methyl 3-(3-(4-chlorobenzyl)-2,6-dioxo-4-(4-(pyrazin-2-oxyl)phenyl)amino)-3,6-dihydropyrimidin-1(2H)-yl)-2,2-dimethylpropionate |
| 5 | 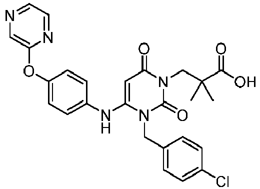 | 3-(3-(4-chlorobenzyl)-2,6-dioxo-4-(4-(pyrazin-2-oxyl)phenyl)amino)-3,6-dihydropyrimidin-1(2H)-yl)-2,2-dimethylpropionic acid |
| 6 | 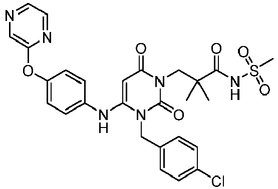 | 3-(3-(4-chlorobenzyl)-2,6-dioxo-4-(4-(pyrazin-2-oxyl)phenyl)amino)-3,6-dihydropyrimidin-1(2H)-yl)-2,2-dimethyl-N-(methylsulfonyl)propionamide group |
| 7 | 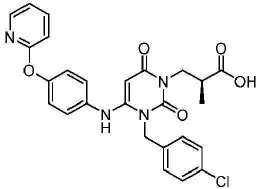 | (S)-3-(3-(4-chlorobenzyl)-2,6-dioxo-4-(4-(pyridin-2-oxyl)phenyl)amino)-3,6-dihydropyrimidin-1(2H)-yl)-2-methylpropionic acid |
| 8 | 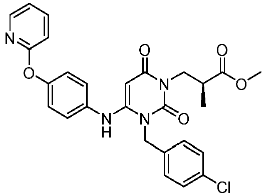 | Methyl 3-(3-(4-chlorobenzyl)-2,6-dioxo-4-(4-(pyridin-2-oxyl)phenyl)amino)-3,6-dihydropyrimidin-1(2H)-yl)-2-methylpropionate |
| 9 | 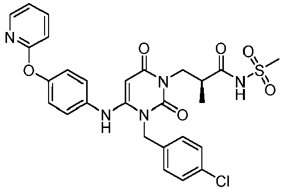 | (S)-3-(3-(4-chlorobenzyl)-2,6-dioxo-4-(4-(pyridin-2-oxyl)phenyl)amino)-3,6-dihydropyrimidin-1(2H)-yl)-2-methyl-N-(methylsulfonyl)acrylamide group |
| 10 | 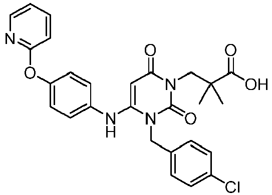 | 3-(3-(4-chlorobenzyl)-2,6-dioxo-4-(4-(pyridin-2-oxyl)phenyl)amino)-3,6-dihydropyrimidin-1(2H)-yl)-2,2-dimethylpropionic acid |
| 11 | 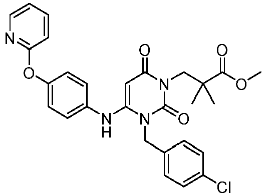 | Methyl 3-(3-(4-chlorobenzyl)-2,6-dioxo-4-(4-(pyridin-2-oxyl)phenyl)amino)-3,6-dihydropyrimidin-1(2H)-yl)-2,2-dimethylpropionate |
| 12 | 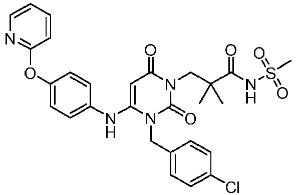 | 3-(3-(4-chlorobenzyl)-2,6-dioxo-4-(4-(pyridin-2-oxyl)phenyl)amino)-3,6-dihydropyrimidin-1(2H)-yl)-2,2-dimethyl-N-(methylsulfonyl)propionamide group |
| 13 | 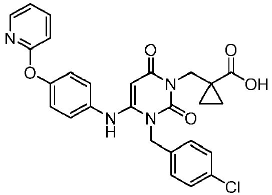 | 1-(3-(4-chlorobenzyl)-2,6-dioxo-4-(4-(pyridin-2-oxyl)phenyl)amino)-3,6-dihydropyrimidinyl-1(2H)-methyl)-cyclopropane-1-carboxylic acid |
| 14 | 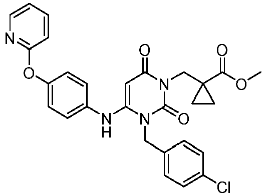 | 1-(3-(4-chlorobenzyl)-2,6-dioxo-4-(4-(pyridin-2-oxyl)phenyl)amino)-3,6-dihydropyrimidinyl-1(2H)-methyl)-cyclopropane-1 -carboxylate |
| 15 | 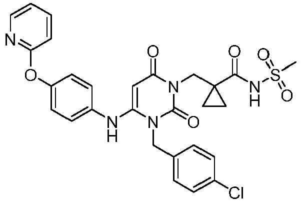 | 1-(3-(4-chlorobenzyl)-2,6-dioxo-4-(4-(pyridin-2-oxyl)phenyl)amino)-3,6-dihydropyrimidin-1(2H)yl)methyl)-N-(methylsulfonyl)cyclopropane-1-carboxamide group |
| 16 | 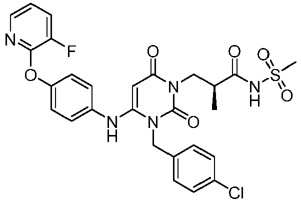 | (S)-3-(3-(4-chlorobenzyl)-4-(4-(3-(3-fluoropyridin-2-yl)oxyphenyl)amino)-2,6-dioxo-3,6-dihydropyrimidin-1(2H)yl)-2-methyl-N-(methylsulfonyl)propionamide group |
| 17 | 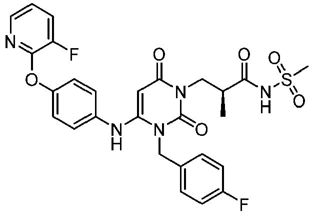 | (S)-3-(3-(4-fluorobenzyl)-4-(4-(3-fluoropyridin-2-yl)phenyl)amino)-2,6-dioxo-3,6-dihydropyrimidin-1 (2H)yl)-2-methyl-N-(methylsulfonyl)propionamide group |
| 18 | 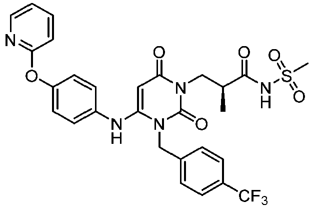 | (S)-3-(2,6-dioxo-4-(4-(pyridin-2-oxyl)phenyl)amino)-3-(4-(trifluoromethyl)benzyl)-3,6-dihydropyrimidin-1(2H)yl)-2-methyl-N-(methylsulfonyl)propionamide group |
| 19 | 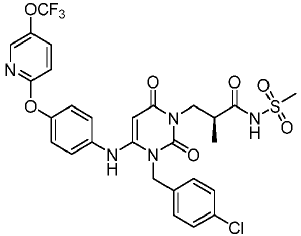 | (S)-3-(3-(4-chlorobenzyl)-2,6-dioxo-4-(4-(5-((5-trifluoromethoxy)pyridin-2-yl)oxyphenyl)amino)-3,6-dihydropyrimidin-1(2H)-yl)-2-methyl-N-(sulfonyl)propionamide group |
| 20 | 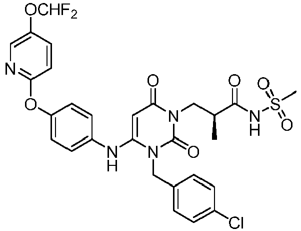 | (S)-3-(3-(4-chlorobenzyl)-2,6-dioxo-4-(4-(5-((5-difluoromethoxy)pyridin-2-yl)oxyphenyl)amino)-3,6-dihydropyrimidin-1(2H)-yl)-2-methyl-N-(sulfonyl)propionamide group |
| 21 | 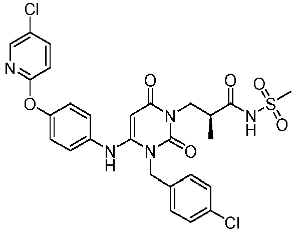 | (S)-3-(3-(4-chlorobenzyl)-4-(4-(5-chloropyridin-2-yl)phenyl)amino)-2,6-dioxo-3,6-dihydropyrimidin-1(2H)yl)-2-methyl-N-(methylsulfonyl)propionamide group |
| 22 | 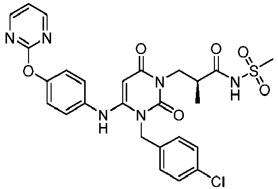 | (S)-3-(3-(4-chlorobenzyl)-2,6-dioxo-4-(4-(pyrimidin-2-oxyl)phenyl)amino)-3,6-dihydropyrimidin-1(2H)-yl)-2-methyl-N-(methylsulfonyl)propionamide group |
| 23 | 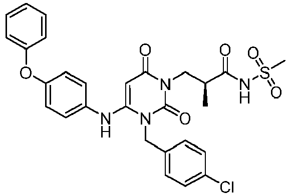 | (S)-3-(3-(4-chlorobenzyl)-2,6-dioxo-4-(4-phenoxyphenyl)amino)-3,6-dihydropyrimidin-1(2H)-yl)-2-methyl-N-(methylsulfonyl)propionamide group |
| 24 | 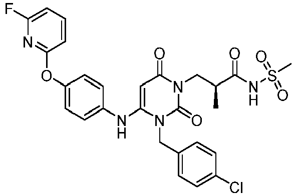 | (S)-3-(3-(4-chlorobenzyl)-4-(4-(6-fluoropyridin-2-oxyl)phenyl)amino)-2,6-dioxo-3,6-dihydropyrimidin-1(2H)yl)-2-methyl-N-(methylsulfonyl)propionamide group |
| 25 | 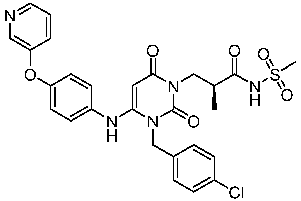 | (S)-3-(3-(4-chlorobenzyl)-2,6-dioxo-4-(4-(pyridin-3-oxyl)phenyl)amino)-3,6-dihydropyrimidin-1(2H)-yl)-2-methyl-N-(methylsulfonyl)propionamide group |
| 26 | 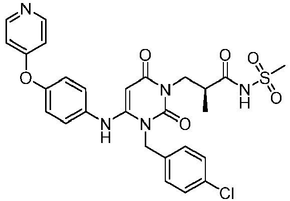 | (S)-3-(3-(4-chlorobenzyl)-2,6-dioxo-4-(4-(pyridin-4-oxyl)phenyl)amino)-3,6-dihydropyrimidin-1(2H)-yl)-2-methyl-N-(methylsulfonyl)propionamide group |
| 27 | 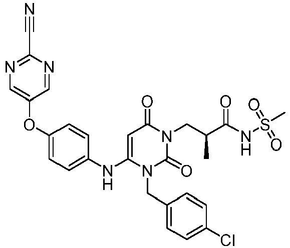 | (S)-3-(3-(4-chlorobenzyl)-4-(4-(2-cyanopyrimidin-5-oxyl)phenyl)-amino)-2,6-dioxo-3,6-dihydropyrimidin-1(2H)-yl)-2-methyl-N-(methylsulfonyl)propionamide group |
| 28 | 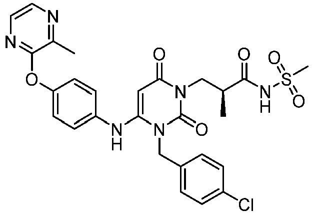 | (S)-3-(3-(4-chlorobenzyl)-4-(4-(3-(3-methylpyrazin-2-oxyl)phenyl)amino)-2,6-dioxo-3,6-dihydropyrimidin-1(2H)-yl)-2-methyl-N-(methylsulfonyl)propionamide group |
| 29 | 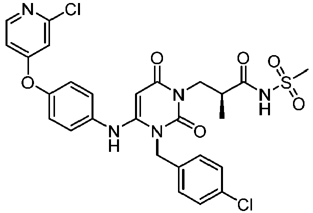 | (S)-3-(3-(4-chlorobenzyl)-4-(4-(2-chloropyridin-4-oxyl)phenyl)amino)-2,6-dioxo-3,6-dihydropyrimidin-1(2H)yl)-2-methyl-N-(methylsulfonyl)propionamide group |
| 30 | 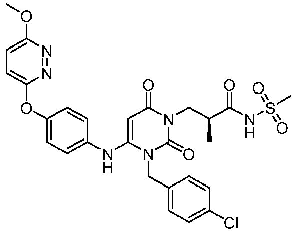 | (S)-3-(3-(4-chlorobenzyl)-4-(4-(6-methoxypyrimidin-3-oxyl)phenyl)-2,6-dioxo-3,6-dihydropyrimidin-1(2H)-yl)-2-methyl-N-(methylsulfonyl)propionamide group |
| 31 | 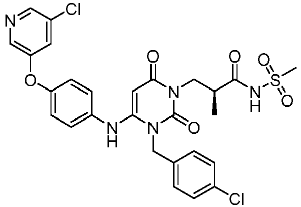 | (S)-3-(3-(4-chlorobenzyl)-4-(4-(5-chloropyridin-3-oxyl)phenyl)amino)-2,6-dioxo-3,6-dihydropyrimidin-1(2H)yl)-2-methyl-N-(methylsulfonyl)propionamide group |
| 32 | 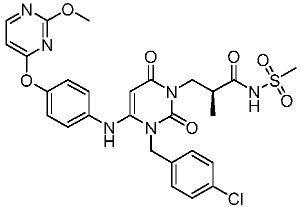 | (S)-3-(3-(4-chlorobenzyl)-4-(4-(2-methoxypyrimidin-4-oxyl)phenyl)-amino)-2,6-dioxo-3,6-dihydropyrimidin-1(2H)-yl)-2-methyl-N-(methylsulfonyl)propionamide group |
| 33 | 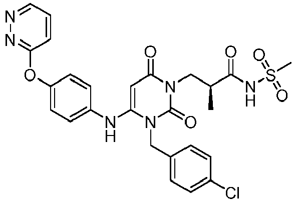 | (S)-3-(3-(4-chlorobenzyl)-2,6-dioxo-4-(4-(pyridazin-3-oxyl)phenyl)amino)-3,6-dihydropyrimidin-1(2H)-yl)-2-methyl-N-(methylsulfonyl)propionamide group |
| 34 | 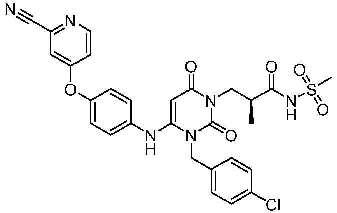 | (S)-3-(3-(4-chlorobenzyl)-4-(4-(2-cyanopyridin-4-oxyl)phenyl)amino)-2,6-dioxo-3,6-dihydropyrimidin-1(2H)-yl)-2-methyl-N-(methylsulfonyl)propionamide group |
| 35 | 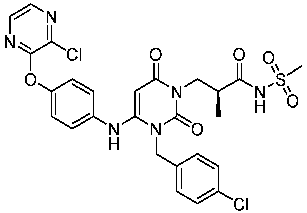 | (S)-3-(3-(4-chlorobenzyl)-4-(4-(3-chloropyrazin-2-oxyl)phenyl)amino)-2,6-dioxo-3,6-dihydropyrimidin-1(2H)yl)-2-methyl-N-(methylsulfonyl)propionamide group |
| 36 | 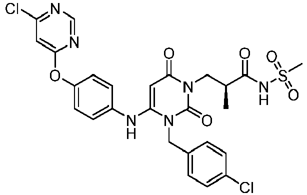 | (S)-3-(3-(4-chlorobenzyl)-4-(4-(6-chloropyrimidin-4-oxyl)phenyl)amino)-2,6-dioxo-3,6-dihydropyrimidin-1(2H)yl)-2-methyl-N-(methylsulfonyl)propionamide group |
| 37 | 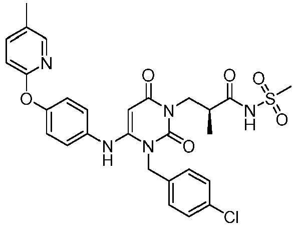 | (S)-3-(3-(4-chlorobenzyl)-4-(4-(5-methylpyridin-2-oxyl)phenyl)amino)-2,6-dioxo-3,6-dihydropyrimidin-1(2H)yl)-2-methyl-N-(methylsulfonyl)propionamide group |
| 38 | 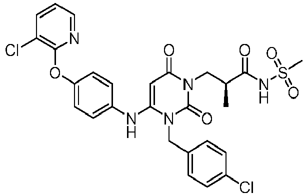 | (S)-3-(3-(4-chlorobenzyl)-4-(4-(3-chloropyridin-2-oxyl)phenyl)amino)-2,6-dioxo-3,6-dihydropyrimidin-1(2H)yl)-2-methyl-N-(methylsulfonyl)propionamide group |
| 39 | 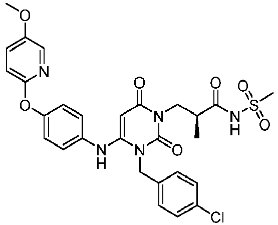 | (S)-3-(3-(4-chlorobenzyl)-4-(4-(5-methoxypyridin-2-oxyl)phenyl)amino)-2,6-dioxo-3,6-dihydropyrimidin-1(2H)yl)-2-methyl-N-(methylsulfonyl)propionamide group |
| 40 | 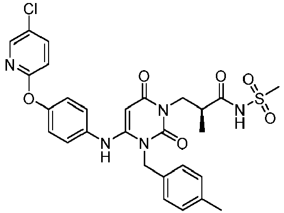 | (S)-3-(4-(4-((5-chloropyridin-2-oxyl)phenyl)amino)-3-(4-methylbenzyl)-2,6-dioxo-3,6-dihydropyrimidin-1(2H)yl)-2-methyl-N-(methylsulfonyl)propionamide group |
| 41 | 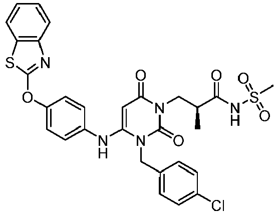 | (S)-3-(4-(4-(4-(benzo[D]thiazol-2-oxyl)phenyl)amino)-3-(4-chlorobenzyl)-2,6-dioxo-3,6-dihydropyrimidin-1(2H)yl)-2-methyl-N-(methylsulfonyl)propionamide group |
| 42 | 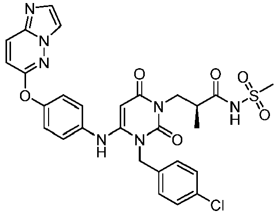 | (S)-3-(3-(4-chlorobenzyl)-4-(4-(imidazo[1,2-B]pyridazin-6-oxyl)phenyl)amino)-2,6-dioxo-3,6-dihydropyrimidin-1(2H)-yl)-2-methyl-N-(sulfonyl)propionamide group |
| 43 | 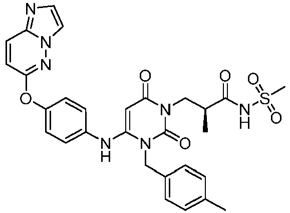 | (S)-3-(4-(4-(4-(imidazo[1,2-B]pyridazin-6-oxylphenyl)amino)-3-(4-methylbenzyl)-2,6-dioxo-3,6-dihydropyrimidin-1(2H)-yl)-2-methyl-N-(sulfonyl)propionamide group |
| 44 | 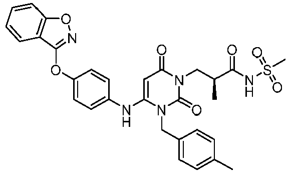 | (S)-3-(4-(4-(benzo[D]isoxazol-3-oxyl)phenyl)amino)-3-(4-methylbenzyl)-2,6-dioxo-3,6-dihydropyrimidin-1(2H)yl)-2-methyl-N-(methylsulfonyl)propionamide group |
| 45 | 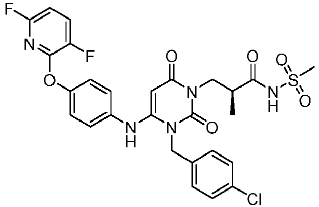 | (S)-3-(4-(4-(3,6-difluoropyridin-2-oxyl)phenyl)amino)-3-(4-chlorobenzyl)-2,6-dioxo-3,6-dihydropyrimidin-1(2H)yl)-2-methyl-N-(sulfonyl)propionamide group |
| 46 | 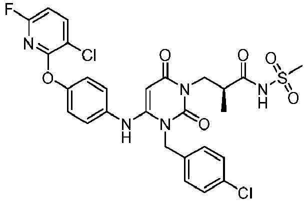 | (S)-3-(4-(4-(3-chloro-6-fluoropyridin-2-oxyl)phenyl)amino)-3-(4-chlorobenzyl)-2,6-dioxo-3,6-dihydropyrimidin-1(2H)yl)-2-methyl-N-(sulfonyl)propionamide group |
| 47 | 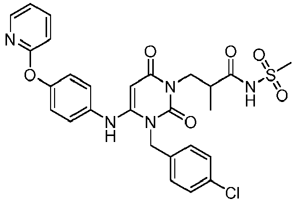 | 3-(3-(4-chlorobenzyl)-2,6-dioxo-4-(4-(pyridin-2-oxyl)phenyl)amino)-3,6-dihydropyrimidin-1(2H)-yl)-2-methyl-N-(methylsulfonyl)acrylamide group |
| 48 | 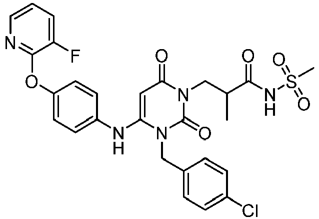 | 3-(3-(4-chlorobenzyl)-4-(4-(3-(3-fluoropyridin-2-oxyl)oxyphenyl)amino)-2,6-dioxo-3,6-dihydropyrimidin-1 (2H)yl)-2-methyl-N-(methylsulfonyl)propionamide group |
| 49 | 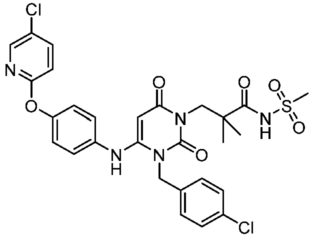 | 3-(3-(4-chlorobenzyl)-4-(4-(5-chloropyridin-2-oxyl)phenyl)amino)-2,6-dioxo-3,6-dihydropyrimidin-1(2H)-yl)-2,2-dimethyl-N-(methylsulfonyl)propionamide group |
| 50 | 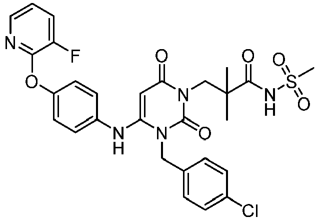 | 3-(3-(4-chlorobenzyl)-4-(4-(3-fluoropyridin-2-oxyl)phenyl)amino)-2,6-dioxo-3,6-dihydropyrimidin-1(2H)-yl)-2,2-dimethyl-N-(methylsulfonyl)propionamide group |
| 51 | 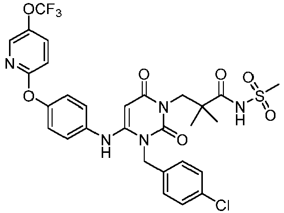 | 3-(3-(4-chlorobenzyl)-4-(4-(5-trifluorooxy-pyridin-2-oxyl)phenyl)amino)-2,6-dioxo-3,6-dihydropyrimidin-1(2H)-yl)-2,2-dimethyl-N-(methylsulfonyl)propionamide group |
| 52 | 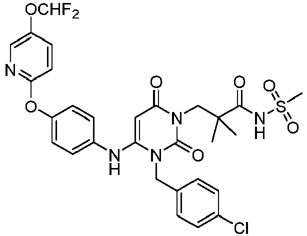 | 3-(3-(4-chlorobenzyl)-4-(4-(5-difluorooxy-pyridin-2-oxyl)phenyl)amino)-2,6-dioxo-3,6-dihydropyrimidin-1(2H)-yl)-2,2-dimethyl-N-(methylsulfonyl)propionamide group |
| 53 | 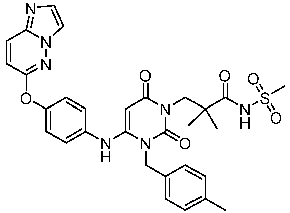 | 3-(4-(4-(4-(imidazo[1,2-B]pyridazin-6-oxyl)phenyl)amino)-3-(4-methylbenzyl)-2,6-dioxo-3,6-dihydropyrimidin-1(2H)yl)-2,2-dimethyl-N-(methylsulfonyl)propionamide group |
| 54 | 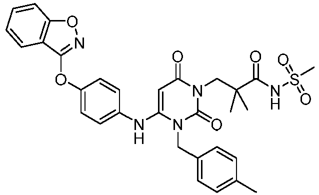 | 3-(4-(4-(benzo[D]isoxazol-3-oxyl)phenyl)amino)-3-(4-methylbenzyl)-2,6-dioxo-3,6-dihydropyrimidin-1(2H)yl)-2,2-dimethyl-N-(methylsulfonyl)propionamide group |
| 55 | 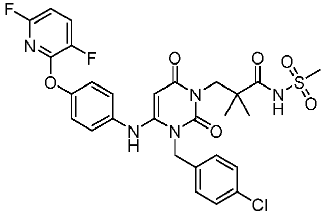 | 3-(3-(4-chlorobenzyl)-4-(4-(3,6-difluoropyridin-2-oxyl)phenyl)-amino)-2,6-dioxo-3,6-dihydropyrimidin-1(2H)-yl)-2,2-dimethyl-N-(methylsulfonyl)propionamide group |
| 56 | 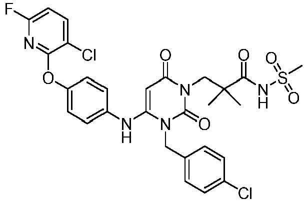 | 3-(4-(4-(3-chloro-6-fluoropyridin-2-oxyl)phenyl)amino)-3-(4-chlorobenzyl)-2,6-dioxo-3,6-dihydropyrimidin-1(2H)yl)-2,2-dimethyl-N-(methylsulfonyl)propionamide group |
| 57 | 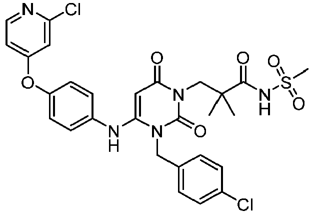 | 3-(3-(4-chlorobenzyl)-4-(4-(2-chloropyridin-4-oxyl)phenyl)amino)-2,6-dioxo-3,6-dihydropyrimidin-1(2H)yl)-2,2-dimethyl-N-(methylsulfonyl)propionamide group |
| 58 | 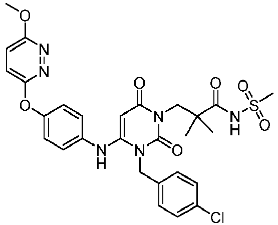 | 3-(3-(4-chlorobenzyl)-4-(4-(6-methoxypyrimidin-3-oxyl)phenyl)amino)-2,6-dioxo-3,6-dihydropyrimidin-1(2H)-yl)-2,2-dimethyl-N-(methylsulfonyl)propionamide group |
| 59 | 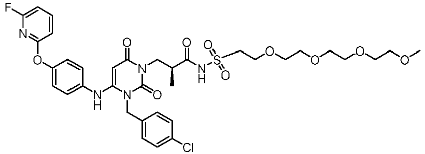 | (S)-N-(2,5,8,11-tetraoxotridecyl-13-yl)sulfonyl)-3-(3-(4-chlorobenzyl)-4-(4-(6-fluoropyridin-2-oxyl)phenyl)amino)-2,6-dioxin-3,6-dihydropyrimidinyl-1(2H)-2-methylpropionamide group |
| 60 | | (S)-3-(3-(4-chlorobenzyl)-4-(4-(6-fluoropyridin-2-oxyl)phenyl)amino)-2,6-dioxo-3,6-dihydropyrimidin-1(2H)yl)-N-(hexadecylsulfonyl)-2-methylpropionamide group |

In some embodiments of the present invention, the hydrogen in the above compounds may be substituted by one or more deuterium.

The method for preparing the compound or a salt, a solvate, an isomer, a metabolite, a nitrogen oxide, or a prodrug thereof provided by the present invention comprises the following steps:
when R¹ is methoxy:
   Step I: subjecting compound **a** and compound **k** under an alkaline condition to a substitution reaction to produce compound **b;**
   Step II: subjecting intermediate compounds **c** and **d** in the presence of an organic alkaline or an inorganic alkaline to a substitution reaction to produce **e;**
   Step III: subjecting intermediate compounds **e** and **f** to a Mitsunobu reaction to produce an intermediate **g;**
   Step IV: subjecting intermediate compounds **g** and **b** to a coupling reaction to produce an intermediate **h;** 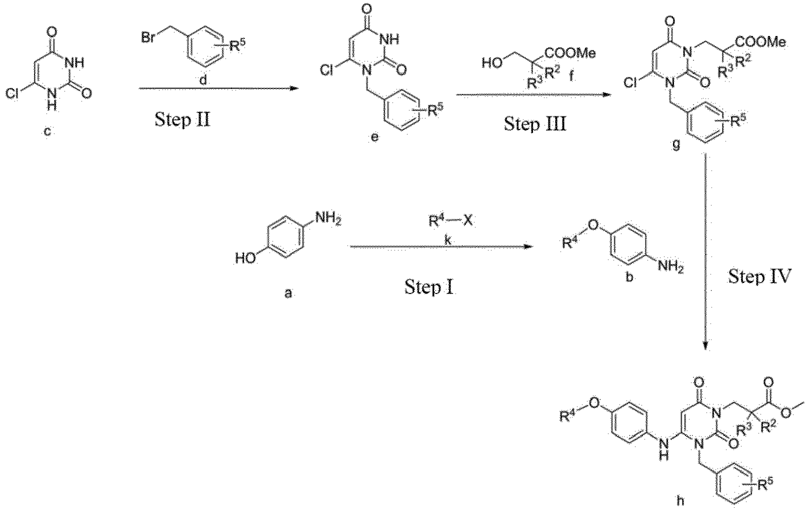
   wherein R², R³, R⁴ and R⁵ are as defined in any of the above, and X is halogen;
   preferably, when R¹ is hydroxy, the method further comprises Step V: subjecting compound **h** under the catalysis of an inorganic alkaline or an acid to a hydrolysis reaction to produce compound **j;** 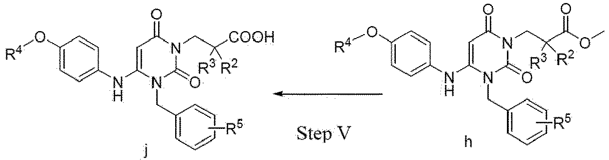
   wherein R², R³, R⁴ and R⁵ are as defined above;
   more preferably, when R¹ is selected from a substituted or unsubstituted alkoxy, a substituted or unsubstituted sulfonamide group, a substituted or unsubstituted heterocyclic epoxy group, and a substituted or unsubstituted aryloxy, and R¹ is not methoxy, the method further comprises Step VI: subjecting compound **j** and an alcohol-, sulfonamide group- or phenol-based compound having an R¹ group to a condensation or esterification reaction to produce a compound of formula I; 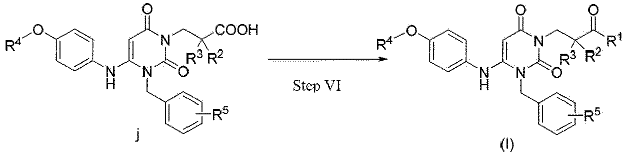
   wherein R¹, R², R³, R⁴ and R⁵ are as defined above.

Provided is the use of the compound or a salt, a solvate, an isomer, a metabolite, a nitrogen oxide, or a prodrug thereof according to the present invention in the manufacture of a medicament for treating or preventing a disease associated with a P2X3 and/or P2X2/3 receptor, preferably for treating or preventing a respiratory disease, and more preferably for treating or preventing cough, asthma, pain, or sleep apnea.

The present invention has the following beneficial effects over the prior art.

The dihydropyrimidine-based compound of the present invention has good P2X3 receptor antagonism and improved safety. Tests have shown that the cough suppressant effect of the present invention is stronger than that of the positive drugs and has a longer duration of action. The inhibitory effect on the P2X3 receptor is good and , and it has almost no impact on the sense of taste in mice.

The dihydropyrimidine-based compound of the present invention is useful for treating or preventing P2X3 and/or /P2X2/3 receptor-related diseases with improved efficacy and safety.

### Detailed Description of the Invention

Hereinafter the present invention will be described in further details in connection with the examples and test examples. The examples and test examples of the present invention are only used to illustrate the technical solutions of the present invention, and not to limit the present invention, and any equivalent substitutions in the related field in accordance with the disclosure of the present invention are within the protection scope of the present invention.

Compound structures were determined by ¹H NMR or LC-MS.

The LC-MS instrument is an Agilent G6120B (in connection with an Agilent 1260); the ¹H NMR instrument is a Bruker AVANCE-400 or a Bruker AVANCE-800, and the ¹H NMR shift (δ) is given in parts per million (ppm), with DMSO as the test solvent, and tetramethylsilane (TMS) as the internal standard. Chemical shifts are given in a unit of 10⁻⁶ (ppm).

The term "room temperature" in the present invention refers to a temperature of 10-25°C.

**Example 1:** Preparation of methyl (S)-3-(3-(4-chlorobenzyl)-2,6-dioxo-4-(4-(pyrazin-2-oxyl)phenyl)amino)-3,6-dihydropyrimidin-1(2H)-yl)-2-methylpropionate (Compound 1)

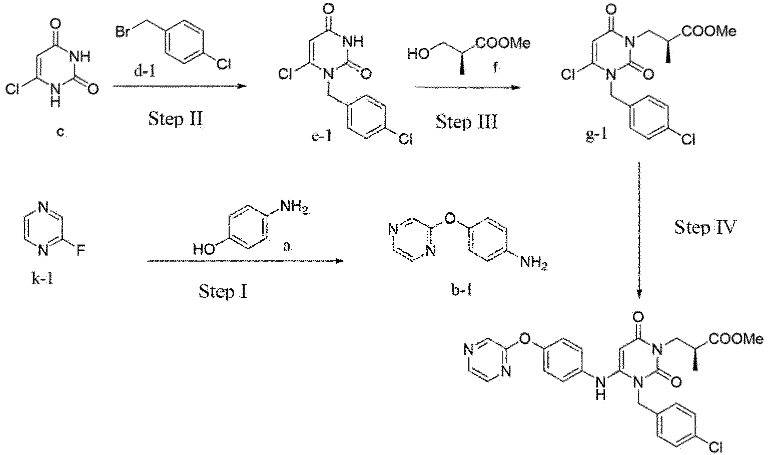

### Step I: preparation of 4-(pyrazin-2-oxyl)aniline (Compound b-1)

2-Fluoropyrazine (50.0g, 0.52mol) and p-aminophenol (53.5g, 0.49 mol) were dissolved in dimethyl sulfoxide (360ml), into which cesium carbonate (320g, 0.98mol) was added to obtain a reaction mixture. The reaction mixture was stirred at a constant speed under mechanical stirring. Then, the temperature of the reaction system was raised to 80°C for a reaction for 2-3h. The reaction progress was monitored by TLC(thin-layer chromatography), and after the reaction was complete, the reaction mixture was added into three times volume of water (about 1L) while stirring was maintained. Thereafter, the resultant was extracted three times with ethyl acetate, and a crude product was combined after it has been concentrated after drying the ethyl acetate. The crude product was prepared into a slurry with 500 ml water for 1h, filtrated, and dried in an air-drying oven to obtain 4-(pyrazin-2-oxyl)aniline (92.8g, brown granular solid), yield: 96.8%.

ESI-MS: *m*/*z* = 187.1 (M+H) ⁺.

### Step II: preparation of 6-chloro-1-(4-chlorobenzyl)pyrimidine-2,4 (1H,3H)-dione (Compound e-1)

6-Chlorouracil (36.8g, 0.25mol) and 4-chlorobenzyl bromide (52.5g, 0.256mol) were mixed and dissolved in 300ml DMF, and DIPEA (96.9g, 0.75mol) was added dropwise thereto to conduct a reaction for 3-5h while maintained at 30°C. The reaction progress was monitored by TLC, and after the reaction was complete, the reaction mixture was added into three times volume of water. The precipitated solid was filtered after wash and dried, and the filter cake was prepared into a slurry with 300ml ethyl acetate. The resulting solid upon filtration was dried in an air-drying oven to obtain 6-chloro-1-(4-chlorobenzyl)pyrimidine-2,4 (1H,3H)-dione (Compound e-1) (51.9g, white solid), yield: 76.6%, purity: 99.26%.

ESI-MS: m/z=271.0 (M+H)⁺.

### Step III: preparation of methyl (S)-3-(4-chloro-3-(4-chlorobenzyl)-2,6-dioxo-3,6-dihydropyrimidin-1(2H)-yl)-2-methylpropionate (Compound g-1)

Compound e-1 (27.1g, 0.1mol), methyl (S)-(+)-3-hydroxy-2-methylpropionate (11.8g, 0.1mol) and triphenylphosphine (52.4g, 0.2mol) was dissolved in 300ml anhydrous tetrahydrofuran until it was clear, and after replacing the air in the reaction system with argon, the reaction system was cooled in an iced water bath. Diisopropyl azodicarboxylate (40.4g, 0.2mol) was slowly added dropwise at a constant speed while stirring was maintained, and after the addition was completed in 30min, the reaction was conducted at rt. 2-3 hours later the reaction progress was monitored by TLC, and after the reaction was complete, the reaction solution was quenched in 500ml water, extracted three times with 30ml ethyl acetate, and an oily crude product was obtained after drying and concentrating the organic solution. The oily crude product was dispersed in a mixed solvent of 100ml ethyl acetate and 500ml petroleum ether, and a large amount of triphenylphosphine was precipitated. The triphenylphosphine was removed upon filtration, and the mother liquid was concentration and purified by chromatography to obtain methyl (S)-3-(4-chloro-3-(4-chlorobenzyl)-2,6-dioxo-3,6-dihydropyrimidin-1(2H)-yl)-2-methylpropionate (32.1g, white solid), yield: 86.6%, purity: 98.71%.

ESI-MS:m/z=371.1 (M+H)⁺.

### Step IV: preparation of methyl (S)-3-(3-(4-chlorobenzyl)-2,6-dioxo-4-(4-(pyrazin-2-oxyl)phenyl)amino)-3,6-dihydropyrimidin-1(2H)-yl)-2-methylpropionate (Compound 1)

Compound g-1 (3.71g, 0.01mol), Compound b-1 (1.87g, 0.01mol), xant-phos (868mg, 1.5mmol), palladium acetate (337mg, 1.5mmol) and potassium phosphate (4.24g, 0.02mol) were mixed and then dissolved in 30ml dioxane, and the air in the reaction flask was replaced with argon. The reaction was conducted under argon protection, and the reaction mixture was heated to 80°C in an oil bath for a reaction for 1-2h. The reaction was monitored by TLC until Compound g-1 was completely depleted, and the reaction mixture was evaporated under reduced pressure to remove dioxane and then extracted three times with 100 ml ethyl acetate and 100 ml water. The ethyl acetate phase was concentrated and purified by column chromatography to obtain methyl (S)-3-(3-(4-chlorobenzyl)-2,6-dioxo-4-(4-(pyrazin-2-oxyl)phenyl)amino)-3,6-dihydropyrimidin-1(2H)-yl)-2-methylpropionate (Compound 1) 4.56g (tawny foamy solid), yield: 87.3%, purity: 96.82%.

ESI-MS: m/z=522.2 (M+H)⁺.

¹H NMR (400 MHz, DMSO-d6) δ 8.65 (s, 1H), δ 8.17 (s, 1H), 7.55-7.48 (m, 1H), 7.46-7.37 (m, 1H), 7.35-7.26 (m, 2H), 7.16 (m, 4H), 7.14-7.11 (m, 1H), 7.04 (d, J = 8.3 Hz, 1H), 5.28 (s, 2H), 4.61 (s, 1H), 3.88 (m, 2H), 3.46 (s, 3H), 2.76 (m, 1H), 0.99 (m, 3H).

### Example 2: Preparation of S-3-(3-(4-chlorobenzyl)-2,6-dioxo-4-(4-(pyrazin-2-oxyl)phenyl)amino)-3,6-dihydropyrimidin-1(2H)-yl)-2-methylpropionic acid (Compound 2)

Methyl (S)-3-(3-(4-chlorobenzyl)-2,6-dioxo-4-(4-(pyrazin-2-oxyl)phenyl)-amino)-3,6-dihydropyrimidin-1(2H)-yl)-2-methylpropionate (522mg, 1.0mmol) prepared according to the method in Example 1 was dissolved in a mixed solvent of methanol (3ml) and tetrahydrofuran (3ml), and while the temperature was maintained at around 10°C, an aqueous solution (3ml) of lithium hydroxide (168mg, 4mmol) was added thereto to obtain a reaction mixture. The reaction mixture was reacted at RT overnight. The reaction progress was monitored by TLC, and after the reaction was complete, the reaction mixture was purified by column chromatography to obtain S-3-(3-(4-chlorobenzyl)-2,6-dioxo-4-(4-(pyrazin-2-oxyl)phenyl)amino)-3,6-dihydropyrimidin-1(2H)-yl)-2-methylpropionic acid (388mg, off-white solid), yield: 76.5%, purity: 98.62%.

ESI-MS: *m*/*z* = 508.1 (M+H)⁺.

¹H NMR (400 MHz, DMSO-d6) δ 12.11 (s, 1H), δ 8.85 (s, 1H), δ 8.18 (s, 1H), 7.56-7.49 (m, 1H), 7.48-7.39 (m, 1H), 7.35-7.26 (m, 2H), 7.16 (m, 4H), 7.14-7.11 (m, 1H), 7.04 (d, J = 8.3 Hz, 1H), 5.30 (s, 2H), 4.62 (s, 1H), 4.06-3.79 (m, 2H), 2.75 (m, 1H), 0.98 (m, 3H).

### Example 3: Preparation of (S)-3-(3-(4-chlorobenzyl)-2,6-dioxo-4-(4-(pyrazin-2-oxyl)phenyl)amino)-3,6-dihydropyrimidin-1(2H)-yl)-2-methyl-N-(methylsulfonyl)propionamide group (Compound 3)

S-3-(3-(4-chlorobenzyl)-2,6-dioxo-4-(4-(pyrazin-2-oxyl)phenyl)amino)-3,6-dihydropyrimidin-1(2H)-yl)-2-methylpropionic acid (Compound 2) (288mg, 0.57mmol) prepared according to the method in Example 2, DIPEA (183mg, 1.42mmol) and EDCI (130.8mg, 0.68mmol) were dissolved in dichloromethane (5ml) and stirred for 15min, and methanesulfonamide group (64.7mg, 0.68mmol) and DMAP (83mg, 0.68mmol) were then added and a reaction was conducted at room temperature for 2-3h. The reaction progress was monitored by TLC, and after the reaction was complete, the reaction mixture was washed with water, extracted three times with dichloromethane, and combined, dried, concentrated and purified by column chromatography to obtain (S)-3-(3-(4-chlorobenzyl)-2,6-dioxo-4-(4-(pyrazin-2-oxyl)phenyl)amino)-3,6-dihydropyrimidin-1(2H)-yl)-2-methyl-N-(methylsulfonyl)propionamide group (198mg, )yield: 59.6%, purity: 99.87%.

ESI-MS: *m*/*z* = 585.1 (M+H) ⁺.

¹H NMR (400 MHz, DMSO-d6) δ 11.31 (s, 1H), δ 8.65 (s, 1H), δ 8.19 (s, 1H), 7.86-7.76 (m, 1H), 7.48-7.39 (m, 1H), 7.35-7.26 (m, 2H), 7.16 (m, 4H), 7.14-7.11 (m, 1H), 7.04 (d, J = 8.3 Hz, 1H), 5.42-5.15 (s, 2H), 4.62 (s, 1H), 3.88 (m, 2H), 3.01 (s, 3H), 2.69 (m, 1H), 0.99 (m, 3H).

**Example 4:** Preparation of methyl 3-(3-(4-chlorobenzyl)-2,6-dioxo-4-(4-(pyrazin-2-oxyl)phenyl)amino)-3,6-dihydropyrimidin-1(2H)-yl)-2,2-dimethylpropionate (Compound 4)

The preparation method of this example was conducted under the same conditions as those in Example 1, with the exception that compound f-1: methyl (S)-3-hydroxy-2-methylpropionate in Step III was replaced with an equimolar of methyl 3-hydroxy-2,2-dimethylpropionate, to obtain a white solid-like methyl 3-(3-(4-chlorobenzyl)-2,6-dioxo-4-(4-(pyrazin-2-oxyl)phenyl)amino)-3,6-dihydropyrimidin-1(2H)-yl)-2,2-dimethylpropionate (Compound 4), yield: 76.5%, purity: 98.78%.

ESI-MS: *m*/*z* = 536.2 (M+H) ⁺.

¹H NMR (400 MHz, DMSO-d6) δ 8.65 (s, 1H), 8.18 (s, 1H), 7.84 (d, J = 8.1 Hz, 1H), 7.42 (d, J = 8.3 Hz, 1H), 7.29 (d, J = 8.4 Hz, 2H), 7.16 (m, 4H), 7.11 (d, J = 6.6 Hz, 1H), 7.03 (d, J = 8.4 Hz, 1H), 5.26 (s, 2H), 4.59 (s, 1H), 3.93 (s, 2H), 3.44 (s, 3H), 1.06 (s, 6H).

**Example 5:** Preparation of 3-(3-(4-chlorobenzyl)-2,6-dioxo-4-(4-(pyrazin-2-oxyl)phenyl)amino)-3,6-dihydropyrimidin-1(2H)-yl)-2,2-dimethylpropionic acid (Compound 5)

The preparation method of this example was conducted under the same conditions as those in Example 2, with the exception that compound f-1: methyl (S)-3-hydroxy-2-methylpropionate in Step III was replaced with an equimolar of methyl 3-hydroxy-2,2-dimethylpropionate, to obtain a white solid-like 3-(3-(4-chlorobenzyl)-2,6-dioxo-4-(4-(pyrazin-2-oxyl)phenyl)amino)-3,6-dihydropyrimidin-1(2H)-yl)-2,2-dimethylpropionic acid (Compound 5), yield: 82.2%, purity: 98.51%.

ESI-MS: *m*/*z* = 522.2 (M+H) ⁺.

¹H NMR (400 MHz, DMSO-d6) δ 12.07 (s, 1H), 8.58 (s, 1H), 8.20 (s, 1H), 7.89-7.79 (m, 1H), 7.46-7.37 (m, 1H), 7.28 (d, J = 8.2 Hz, 2H), 7.13 (m, 4H), 7.15-7.10 (m, 1H), 7.02 (d, J = 8.3 Hz, 1H), 5.28 (s, 2H), 4.60 (s, 1H), 3.99 (s, 2H), 1.03 (s, 6H).

**Example 6:** Preparation of 3-(3-(4-chlorobenzyl)-2,6-dioxo-4-(4-(pyrazin-2-oxyl)phenyl)amino)-3,6-dihydropyrimidin-1(2H)-yl)-2,2-dimethyl-N-(methylsulfonyl)propionamide group (Compound 6)

The preparation method of this example was conducted under the same conditions as those in Example 3, with the exception that compound f-1: methyl (S)-3-hydroxy-2-methylpropionate in Step III was replaced with an equimolar of methyl 3-hydroxy-2,2-dimethylpropionate, to obtain a white solid-like 3-(3-(4-chlorobenzyl)-2,6-dioxo-4-(4-(pyrazin-2-oxyl)phenyl)amino)-3,6-dihydropyrimidin-1(2H)-yl)-2,2-dimethyl-N-(methylsulfonyl)propionamide group (Compound 6), yield: 78.6%, purity: 97.68%.

ESI-MS: *m*/*z* =599.1 (M+H) ⁺.

¹H NMR (400 MHz, DMSO-d6) δ 11.30 (s, 1H), 8.64 (s, 1H), 8.18 (s, 1H), 7.84 (d, J = 8.1 Hz, 1H), 7.42 (d, J = 8.3 Hz, 1H), 7.29 (d, J = 8.4 Hz, 2H), 7.16 (m, 4H), 7.11 (d, J = 6.6 Hz, 1H), 7.03 (d, J = 8.4 Hz, 1H), 5.25 (s, 2H), 4.60 (d, J = 1.6 Hz, 1H), 4.03 (s, 2H), 3.02 (s, 3H), 1.05 (s, 6H).

**Example 7:** Preparation of (S)-3-(3-(4-chlorobenzyl)-2,6-dioxo-4-(4-(pyridin-2-oxyl)phenyl)amino)-3,6-dihydropyrimidin-1(2H)-yl)-2-methylpropionic acid

### (Compound 7)

The preparation method of this example was conducted under the same conditions as those in Example 2, with the exception that 2-fluoropyrazine in Step I was replaced with an equimolar of 2-fluoropyridine, to obtain a white solid-like (S)-3-(3-(4-chlorobenzyl)-2,6-dioxo-4-(4-(pyridin-2-oxyl)phenyl)amino)-3,6-dihydropyrimidin-1(2H)-yl)-2-methylpropionic acid (Compound 7), yield: 78.2%, purity: 98.68%.

ESI-MS: *m*/*z* = 507.1 (M+H) ⁺.

¹H NMR (400 MHz, DMSO-d6) δ 8.16 (dd, J = 5.1, 2.0 Hz, 1H), 7.94-7.80 (m, 1H), 7.48-7.39 (m, 2H), 7.35-7.26 (m, 2H), 7.16 (m, 4H), 7.14-7.11 (m, 1H), 7.04 (d, J = 8.3 Hz, 1H), 5.30 (s, 2H), 4.62 (s, 1H), 4.06-3.79 (m, 2H), 2.75 (m, 1H), 0.98 (m, 3H).

**Example 8:** Preparation of methyl 3-(3-(4-chlorobenzyl)-2,6-dioxo-4-(4-(pyridin-2-oxyl)phenyl)amino)-3,6-dihydropyrimidin-1(2H)-yl)-2-methylpropionate

### (Compound 8)

The preparation method of this example was conducted under the same conditions as those in Example 1, with the exception that 2-fluoropyrazine in Step I was replaced with an equimolar of 2-fluoropyridine, to obtain a white solid-like methyl 3-(3-(4-chlorobenzyl)-2,6-dioxo-4-(4-(pyridin-2-oxyl)phenyl)amino)-3,6-dihydropyrimidin-1(2H)-yl)-2-methylpropionate (Compound 8), yield: 72.8%, purity: 98.56%.

ESI-MS: *m*/*z* = 521.2 (M+H) ⁺.

¹H NMR (400 MHz, DMSO-d6) δ 8.63 (s, 1H), 8.20-8.11 (m, 1H), 7.90-7.79 (m, 1H), 7.48-7.38 (m, 2H), 7.29 (d, J = 8.3 Hz, 2H), 7.21-7.14 (m, 4H), 7.14-7.10 (m, 1H), 7.03 (d, J = 8.3 Hz, 1H), 5.28 (s, 2H), 4.61 (s, 1H), 3.89 (m, 2H), 3.47 (s, 3H), 2.77 (m, 1H), 1.00 (m, 3H).

**Example 9:** Preparation of (S)-3-(3-(4-chlorobenzyl)-2,6-dioxo-4-(4-(pyridin-2-oxyl)phenyl)amino)-3,6-dihydropyrimidin-1(2H)-yl)-2-methyl-N-(methylsulfonyl)acrylamide group (Compound 9)

The preparation method of this example was conducted under the same conditions as those in Example 3, with the exception that 2-fluoropyrazine in Step I was replaced with an equimolar of 2-fluoropyridine, to obtain a white solid-like (S)-3-(3-(4-chlorobenzyl)-2,6-dioxo-4-(4-(pyridin-2-oxyl)phenyl)amino)-3,6-dihydropyrimidin-1(2H)-yl)-2-methyl-N-(methylsulfonyl)acrylamide group (Compound 9), yield: 85.2%, purity: 99.21%.

ESI-MS: *m*/*z* = 584.2 (M+H) ⁺.

¹H NMR (400 MHz, DMSO-d6) δ 11.69 (s, 1H), 8.60 (s, 1H), 8.15 (dd, J = 5.0, 2.0 Hz, 1H), 7.90-7.79 (m, 1H), 7.45-7.37 (m, 2H), 7.30 (d, J = 8.4 Hz, 2H), 7.15 (m, 4H), 7.14-7.09 (m, 1H), 7.03 (d, J = 8.3 Hz, 1H), 5.42-5.15 (m, 2H), 4.62 (s, 1H), 3.88 (m, 2H), 3.01 (s, 3H), 2.69 (m, 1H), 0.98 (m, 3H).

**Example 10:** Preparation of 3-(3-(4-chlorobenzyl)-2,6-dioxo-4-(4-(pyridin-2-oxyl)phenyl)amino)-3,6-dihydropyrimidin-1(2H)-yl)-2,2-dimethylpropionic acid (Compound 10)

The preparation method of this example was conducted under the same conditions as those in Example 2, with the exception that 2-fluoropyrazine in Step I was replaced with an equimolar of 2-fluoropyridine and that methyl (S)-3-hydroxy-2-methylpropionate in Step III was replaced with an equimolar of methyl 3-hydroxy-2,2-dimethylpropionate, to obtain a white solid-like 3-(3-(4-chlorobenzyl)-2,6-dioxo-4-(4-(pyridin-2-oxyl)phenyl)amino)-3,6-dihydropyrimidin-1(2H)-yl)-2,2-dimethylpropionic acid (Compound 10), yield: 79.6%, purity: 98.26%.

ESI-MS: *m*/*z* = 521.2 (M+H) ⁺.

¹H NMR (400 MHz, DMSO-d6) δ 12.08 (s, 1H), 8.59 (s, 1H), 8.14 (dd, J = 4.8, 2.1 Hz, 1H), 7.89-7.79 (m, 1H), 7.46-7.37 (m, 2H), 7.29 (d, J = 8.2 Hz, 2H), 7.15 (m, 4H), 7.14-7.10 (m, 1H), 7.03 (d, J = 8.3 Hz, 1H), 5.27 (s, 2H), 4.60 (s, 1H), 3.98 (s, 2H), 1.02 (s, 6H).

**Example 11:** Preparation of methyl 3-(3-(4-chlorobenzyl)-2,6-dioxo-4-(4-(pyridin-2-oxyl)phenyl)amino)-3,6-dihydropyrimidin-1(2H)-yl)-2,2-dimethylpropionate (Compound 11)

The preparation method of this example was conducted under the same conditions as those in Example 1, with the exception that 2-fluoropyrazine in Step I was replaced with an equimolar of 2-fluoropyridine and that methyl (S)-3-hydroxy-2-methylpropionate in Step III was replaced with an equimolar of methyl 3-hydroxy-2,2-dimethylpropionate, to obtain a white solid-like methyl 3-(3-(4-chlorobenzyl)-2,6-dioxo-4-(4-(pyridin-2-oxyl)phenyl)amino)-3,6-dihydropyrimidin-1(2H)-yl)-2,2-dimethylpropionate (Compound 11), yield: 75.2%, purity: 97.58%.

ESI-MS: *m*/*z* = 535.2 (M+H) ⁺.

¹H NMR (400 MHz, DMSO-d6) δ 8.62 (s, 1H), 8.15 (d, J = 4.7 Hz, 1H), 7.84 (t, J = 8.1 Hz, 1H), 7.42 (d, J = 8.3 Hz, 2H), 7.29 (d, J = 8.4 Hz, 2H), 7.16 (m, 4H), 7.11 (d, J = 6.6 Hz, 1H), 7.03 (d, J = 8.4 Hz, 1H), 5.26 (s, 2H), 4.59 (s, 1H), 3.93 (s, 2H), 3.44 (s, 3H), 1.06 (s, 6H).

**Example 12:** Preparation of 3-(3-(4-chlorobenzyl)-2,6-dioxo-4-(4-(pyridin-2-oxyl)phenyl)amino)-3,6-dihydropyrimidin-1(2H)-yl)-2,2-dimethyl-N-(methylsulfonyl)propionamide group (Compound 12)

The preparation method of this example was conducted under the same conditions as those in Example 3, with the exception that 2-fluoropyrazine in Step I was replaced with an equimolar of 2-fluoropyridine and that methyl (S)-3-hydroxy-2-methylpropionate in Step III was replaced with an equimolar of methyl 3-hydroxy-2,2-dimethylpropionate, to obtain a white solid-like 3-(3-(4-chlorobenzyl)-2,6-dioxo-4-(4-(pyridin-2-oxyl)phenyl)amino)-3,6-dihydropyrimidin-1(2H)-yl)-2,2-dimethyl-N-(methylsulfonyl)propionamide group (Compound 12), yield: 77.8%, purity: 98.98%.

ESI-MS: *m*/*z* = 598.1 (M+H) ⁺.

¹H NMR (400 MHz, DMSO-d6) δ 11.30 (s, 1H), 8.64 (s, 1H), 8.20-8.09 (m, 1H), 7.84 (m, 1H), 7.42 (d, J = 8.6 Hz, 2H), 7.31-7.25 (m, 2H), 7.20-7.13 (m, 4H), 7.13-7.09 (m, 1H), 7.03 (d, J = 8.3 Hz, 1H), 5.25 (s, 2H), 4.60 (s, 1H), 4.03 (s, 2H), 3.02 (s, 3H), 1.05 (s, 6H).

**Example 13:** Preparation of 1-(3-(4-chlorobenzyl)-2,6-dioxo-4-(4-(pyridin-2-oxyl)phenyl)amino)-3,6-dihydropyrimidinyl-1(2H)-methyl)-cyclopropane-1-carboxylic acid (Compound 13)

The preparation method of this example was conducted under the same conditions as those in Example 2, with the exception that 2-fluoropyrazine in Step I was replaced with an equimolar of 2-fluoropyridine and that methyl (S)-3-hydroxy-2-methylpropionate in Step III was replaced with an equimolar of methyl 1-(hydroxymethyl)cyclopropane-1-carboxylate, to obtain a white solid-like 1-(3-(4-chlorobenzyl)-2,6-dioxo-4-(4-(pyridin-2-oxyl)phenyl)amino)-3,6-dihydropyrimidinyl-1(2H)-methyl)-cyclopropane-1-carboxylic acid (Compound 13), yield: 76.6%, purity: 98.53%.

ESI-MS: *m*/*z* = 519.2 (M+H)⁺.

¹H NMR (400 MHz, DMSO-d6) δ 8.14 (dd, J = 4.9, 2.1 Hz, 1H), 7.83 (m, 1H), 7.45-7.37 (m, 2H), 7.29 (d, J = 8.3 Hz, 2H), 7.11 (m, 5H), 7.01 (d, J = 8.3 Hz, 1H), 5.26 (s, 2H), 4.54 (s, 1H), 4.22 (s, 2H), 0.91 (m, 2H), 0.61 (m, 4H).

**Example 14:** Preparation of 1-(3-(4-chlorobenzyl)-2,6-dioxo-4-(4-(pyridin-2-oxyl)phenyl)amino)-3,6-dihydropyrimidinyl-1(2H)-methyl)-cyclopropane-1-carboxylate (Compound 14)

The preparation method of this example was conducted under the same conditions as those in Example 1, with the exception that 2-fluoropyrazine in Step I was replaced with an equimolar of 2-fluoropyridine and that methyl (S)-3-hydroxy-2-methylpropionate in Step III was replaced with an equimolar of methyl 1-(hydroxymethyl)cyclopropane-1-carboxylate, to obtain a white solid-like 1-(3-(4-chlorobenzyl)-2,6-dioxo-4-(4-(pyridin-2-oxyl)phenyl)amino)-3,6-dihydropyrimidinyl-1(2H)-methyl)-cyclopropane-1-carboxylate (Compound 14), yield: 79.2%, purity: 98.26%.

ESI-MS: *m*/*z* = 533.2 (M+H) ⁺.

¹H NMR (400 MHz, DMSO-d6) δ 8.68 (s, 1H), 8.13 (d, J = 4.7 Hz, 1H), 7.83 (t, J = 8.1 Hz, 1H), 7.45-7.38 (m, 2H), 7.29 (d, J = 8.4 Hz, 2H), 7.16 (m, 4H), 7.12 (d, J = 6.6 Hz, 1H), 7.03 (d, J = 8.4 Hz, 1H), 5.26 (s, 2H), 4.54 (s, 1H), 4.03 (s, 2H), 3.42 (s, 3H), 1.07-0.92 (m, 4H).

**Example 15:** Preparation of 1-(3-(4-chlorobenzyl)-2,6-dioxo-4-(4-(pyridin-2-oxyl)phenyl)amino)-3,6-dihydropyrimidin-1(2H)yl)methyl)-N-(methylsulfonyl)cyclopropane-1-carboxamide group (Compound 15)

The preparation method of this example was conducted under the same conditions as those in Example 3, with the exception that 2-fluoropyrazine in Step I was replaced with an equimolar of 2-fluoropyridine and that methyl (S)-3-hydroxy-2-methylpropionate in Step III was replaced with an equimolar of methyl 1-(hydroxymethyl)cyclopropane-1-carboxylate, to obtain a white solid-like 1-(3-(4-chlorobenzyl)-2,6-dioxo-4-(4-(pyridin-2-oxyl)phenyl)amino)-3,6-dihydropyrimidin-1(2H)yl)methyl)-N-(methylsulfonyl)cyclopropane-1-carboxamide group (Compound 15), yield: 75.8%, purity: 98.31%.

ESI-MS: *m*/*z* = 596.1 (M+H) ⁺.

¹H NMR (400 MHz, DMSO-d6) δ 11.30 (s, 1H), 8.64 (s, 1H), 8.15 (dd, J = 5.2, 2.0 Hz, 1H), 7.85 (m, 1H), 7.46-7.38 (m, 2H), 7.31 (d, J = 8.4 Hz, 2H), 7.23-7.15 (m, 4H), 7.15-7.09 (m, 1H), 7.04 (d, J = 8.3 Hz, 1H), 5.28 (s, 2H), 4.65 (s, 1H), 4.13 (s, 2H), 3.12 (s, 3H), 1.07-0.91 (m, 4H).

**Example 16:** Preparation of (S)-3-(3-(4-chlorobenzyl)-4-(4-(3-(3-fluoropyridin-2-yl)oxyphenyl)amino)-2,6-dioxo-3,6-dihydropyrimidin-1(2H)yl)-2-methyl-N-(methylsulfonyl)propionamide group (Compound 16)

The preparation method of this example was conducted under the same conditions as those in Example 3, with the exception that 2-fluoropyrazine in Step I was replaced with an equimolar of 2,2-difluoropyridine, to obtain a white solid-like (S)-3-(3-(4-chlorobenzyl)-4-(4-(3-(3-fluoropyridin-2-yl)oxyphenyl)amino)-2,6-dioxo-3,6-dihydropyrimidin-1(2H)yl)-2-methyl-N-(methylsulfonyl)propionamide group (Compound 16), yield: 78.5%, purity: 98.89%.

ESI-MS: *m*/*z* = 602.1 (M+H) ⁺.

¹H NMR (400 MHz, DMSO-d6) δ 11.30 (s, 1H), 8.65 (s, 1H), 8.03 (q, J = 8.2 Hz, 1H), 7.44 (d, J = 8.2 Hz, 2H), 7.32 (d, J = 8.2 Hz, 2H), 7.22 (m, 4H), 6.95 (dd, J = 8.0, 1.7 Hz, 1H), 6.90 (dd, J = 7.9, 2.5 Hz, 1H), 5.31 (s, 2H), 4.66 (s, 1H), 4.08-3.80 (m, 2H), 3.01 (s, 3H), 2.73 (m, 1H), 0.99 (m, 3H).

**Example 17:** Preparation of (S)-3-(3-(4-fluorobenzyl)-4-(4-(3-fluoropyridin-2-yl)phenyl)amino)-2,6-dioxo-3,6-dihydropyrimidin-1(2H)yl)-2-methyl-N-(methylsulfonyl)propionamide group (Compound 17)

The preparation method of this example was conducted under the same conditions as those in Example 3, with the exception that 2-fluoropyrazine in Step I was replaced with an equimolar of 2,3-difluoropyridine and that 1-(bromomethyl)-4-chlorobenzene in Step II was replaced with an equimolar of 1-(bromomethyl)-4-fluorobenzene, to obtain a white solid-like (S)-3-(3-(4-fluorobenzyl)-4-(4-(3-fluoropyridin-2-yl)phenyl)amino)-2,6-dioxo-3,6-dihydropyrimidin-1(2H)yl)-2-methyl-N-(methylsulfonyl)propionamide group (Compound 17), yield: 78.9%, purity: 99.12%.

ESI-MS: *m*/*z* = 586.2 (M+H) ⁺.

¹H NMR (400 MHz, DMSO-d6) δ 11.30 (s, 1H), 8.65 (s, 1H), 8.03 (q, J = 8.2 Hz, 1H), 7.44 (d, J = 8.2 Hz, 2H), 7.39 (m, 2H), 7.32 (d, J = 8.2 Hz, 2H), 7.08 (m, 2H), 6.95 (dd, J = 8.0, 1.7 Hz, 1H), 6.90 (dd, J = 7.9, 2.5 Hz, 1H), 5.31 (s, 2H), 4.66 (s, 1H), 4.08-3.80 (m, 2H), 3.01 (s, 3H), 2.73 (m, 1H), 0.99 (m, 3H).

**Example 18:** Preparation of (S)-3-(2,6-dioxo-4-(4-(pyridin-2-oxyl)phenyl)amino)-3-(4-(trifluoromethyl)benzyl)-3,6-dihydropyrimidin-1(2H)yl)-2-methyl-N-(methylsulfonyl)propionamide group (Compound 18)

The preparation method of this example was conducted under the same conditions as those in Example 3, with the exception that 2-fluoropyrazine in Step I was replaced with an equimolar of 2,3-difluoropyridine and that 1-(bromomethyl)-4-chlorobenzene in Step II was replaced with an equimolar of 1-(bromomethyl)-4-trifluoromethylbenzene, to obtain a white solid-like (S)-3-(2,6-dioxo-4-(4-(pyridin-2-oxyl)phenyl)amino)-3-(4-(trifluoromethyl)benzyl)-3,6-dihydropyrimidin-1(2H)yl)-2-methyl-N-(methylsulfonyl)propionamide group (Compound 18), yield: 88.2%, purity: 98.23%.

ESI-MS: *m*/*z* = 617.2 (M+H) ⁺.

¹H NMR (400 MHz, DMSO-d6) δ 11.69 (s, 1H), 8.60 (s, 1H), 8.13 (dd, J = 5.0, 2.0 Hz, 1H), 7.90-7.79 (m, 1H), 7.58-7.50 (m, 2H), 7.45-7.37 (m, 2H), 7.30 (d, J = 8.4 Hz, 2H), 7.18-7.10 (m, 2H), 7.14-7.09 (m, 1H), 7.03 (d, J = 8.3 Hz, 1H), 5.42-5.15 (m, 2H), 4.63 (s, 1H), 3.86 ( m, 2H), 3.02 (s, 3H), 2.68 (m, 1H), 0.99 (m, 3H).

**Example 19:** Preparation of (S)-3-(3-(4-chlorobenzyl)-2,6-dioxo-4-(4-(5-((5-trifluoromethoxy)pyridin-2-yl)oxyphenyl)amino)-3,6-dihydropyrimidin-1(2H)-yl)-2-methyl-N-(sulfonyl)propionamide group (Compound 19)

The preparation method of this example was conducted under the same conditions as those in Example 3, with the exception that 2-fluoropyrazine in Step I was replaced with an equimolar of 2-fluoro-5-(trifluoromethoxy)pyridine, to obtain a white solid-like (S)-3-(3-(4-chlorobenzyl)-2,6-dioxo-4-(4-(5-((5-trifluoromethoxy)pyridin-2-yl)oxyphenyl)amino)-3,6-dihydropyrimidin-1(2H)-yl)-2-methyl-N-(sulfonyl)propionamide group (Compound 19), yield: 82.1%, purity: 98.68%.

ESI-MS: *m*/*z* = 668.1 (M+H) ⁺.

¹H NMR (400 MHz, DMSO-d6) δ 11.68 (s, 1H), 8.59 (s, 1H), 8.16 (s, 1H), 7.90-7.79 (m, 1H), 7.45-7.37 (m, 2H), 7.30 (d, J = 8.4 Hz, 2H), 7.15 (m, 4H), 7.14-7.09 (m, 1H) , 5.42-5.15 (m, 2H), 4.62 (s, 1H), 3.86 (m, 2H), 3.01 (s, 3H), 2.68 (m, 1H), 0.98 (m, 3H).

**Example 20:** Preparation of (S)-3-(3-(4-chlorobenzyl)-2,6-dioxo-4-(4-(5-((5-difluoromethoxy)pyridin-2-yl)oxyphenyl)amino)-3,6-dihydropyrimidin-1(2H)-yl)-2-methyl-N-(sulfonyl)propionamide group (Compound 20)

The preparation method of this example was conducted under the same conditions as those in Example 3, with the exception that 2-fluoropyrazine in Step I was replaced with an equimolar of 2-fluoro-5-(trifluoromethoxy)pyridine, to obtain a white solid-like (S)-3-(3-(4-chlorobenzyl)-2,6-dioxo-4-(4-(5-((5-difluoromethoxy)pyridin-2-yl)oxyphenyl)amino)-3,6-dihydropyrimidin-1(2H)-yl)-2-methyl-N-(sulfonyl)propionamide group (Compound 20), yield: 79.1%, purity: 98.18%.

ESI-MS: *m*/*z* =650.1 (M+H) ⁺.

¹H NMR (400 MHz, DMSO-d6) δ 11.68 (s, 1H), 8.59 (s, 1H), 8.02 (s, 1H), 7.82-7.73 (m, 1H), 7.52 (s, 1H), 7.45-7.37 (m, 2H), 7.30 (d, J = 8.4 Hz, 2H), 7.15 (m, 4H), 7.11-7.06 (m, 1H), 5.42-5.15 (m, 2H), 4.62 (s, 1H), 3.86 (m, 2H), 3.01 (s, 3H), 2.68 m, 1H), 0.98 (m, 3H).

**Example 21:** Preparation of (S)-3-(3-(4-chlorobenzyl)-4-(4-(5-chloropyridin-2-oxyl)phenyl)amino)-2,6-dioxo-3,6-dihydropyrimidin-1(2H)yl)-2-methyl-N-(methylsulfonyl)propionamide group (Compound 21)

The preparation method of this example was conducted under the same conditions as those in Example 3, with the exception that 2-fluoropyrazine in Step I was replaced with an equimolar of 2-fluoro-5-chloropyridine, to obtain a white solid-like (S)-3-(3-(4-chlorobenzyl)-4-(4-(5-chloropyridin-2-oxyl)phenyl)amino)-2,6-dioxo-3,6-dihydropyrimidin-1(2H)yl)-2-methyl-N-(methylsulfonyl)propionamide group (Compound 21), yield: 85.9%, purity: 98.56%.

ESI-MS: *m*/*z* = 618.1 (M+H) ⁺.

¹H NMR (400 MHz, DMSO-d6) δ 11.66 (s, 1H), 8.58 (s, 1H), 8.18 (s, 1H), 7.90-7.79 (m, 1H), 7.45-7.37 (m, 2H), 7.30 (d, J = 8.4 Hz, 2H), 7.22 (d, J = 8.3 Hz, 1H), 7.13 (m, 4H), 5.42-5.15 (s, 2H), 4.62 (s, 1H), 3.88 (m, 2H), 3.01 (s, 3H), 2.69 (m, 1H), 0.98 (m, 3H).

**Example 22:** Preparation of (S)-3-(3-(4-chlorobenzyl)-2,6-dioxo-4-(4-(pyrimidin-2-oxyl)phenyl)amino)-3,6-dihydropyrimidin-1(2H)-yl)-2-methyl-N-(methylsulfonyl)propionamide group (Compound 22)

The preparation method of this example was conducted under the same conditions as those in Example 3, with the exception that 2-fluoropyrazine in Step I was replaced with an equimolar of 2-fluoropyrimidine, to obtain a white solid-like (S)-3-(3-(4-chlorobenzyl)-2,6-dioxo-4-(4-(pyrimidin-2-oxyl)phenyl)amino)-3,6-dihydropyrimidin-1(2H)-yl)-2-methyl-N-(methylsulfonyl)propionamide group (Compound 22), yield: 81.5%, purity: 99.74%.

ESI-MS: *m*/*z* = 585.1 (M+H) ⁺.

¹H NMR (400 MHz, DMSO-d6) δ 11.69 (s, 1H), 8.60 (s, 1H), 8.28 (dd, J = 5.0, 2.0 Hz, 2H), 7.45-7.37 (m, 2H), 7.30 (d, J = 8.4 Hz, 2H), 7.15 (m, 4H), 7.08-7.01 (m, 1H), 5.42-5.15 (m, 2H), 4.62 (s, 1H), 3.88 (m, 2H), 3.01 (s, 3H), 2.69 (m, 1H), 0.98 (m, 3H) .

**Example 23:** Preparation of (S)-3-(3-(4-chlorobenzyl)-2,6-dioxo-4-(4-phenoxyphenyl)amino)-3,6-dihydropyrimidin-1(2H)-yl)-2-methyl-N-(methylsulfonyl)propionamide group (Compound 23)

The preparation method of this example was conducted under the same conditions as those in Example 3, with the exception that 2-fluoropyrazine in Step I was replaced with an equimolar of 2-fluorobenzene, to obtain a white solid-like (S)-3-(3-(4-chlorobenzyl)-2,6-dioxo-4-(4-phenoxyphenyl)amino)-3,6-dihydropyrimidin-1(2H)-yl)-2-methyl-N-(methylsulfonyl)propionamide group (Compound 23), yield: 78.3%, purity: 98.11%.

ESI-MS: *m*/*z* = 583.1 (M+H)⁺.

¹H NMR (400 MHz, DMSO-d6) δ 11.69 (s, 1H), 8.60 (s, 1H), 8.03 (m, 2H), 7.61-7.53 (m, 2H), 7.30 (d, J = 8.4 Hz, 2H), 7.15 (m, 4H), 7.24-7.19 (m, 1H), 7.03 (d, J = 8.3 Hz, 2H), 5.42-5.15 (s, 2H), 4.62 (s, 1H), 3.88 (m, 2H), 3.03 (s, 3H), 2.69 (m, 1H), 0.98 (m, 3H).

**Example 24:** Preparation of (S)-3-(3-(4-chlorobenzyl)-4-(4-(6-fluoropyridin-2-oxyl)phenyl)amino)-2,6-dioxo-3,6-dihydropyrimidin-1(2H)yl)-2-methyl-N-(methylsulfonyl)propionamide group (Compound 24)

The preparation method of this example was conducted under the same conditions as those in Example 3, with the exception that 2-fluoropyrazine in Step I was replaced with an equimolar of 2,6-difluorobenzene, to obtain a white solid-like (S)-3-(3-(4-chlorobenzyl)-4-(4-(6-fluoropyridin-2-oxyl)phenyl)amino)-2,6-dioxo-3,6-dihydropyrimidin-1(2H)yl)-2-methyl-N-(methylsulfonyl)propionamide group (Compound 24), yield: 78.2%, purity: 98.22%.

ESI-MS: *m*/*z* = 602.1 (M+H) ⁺.

¹H NMR (400 MHz, DMSO-d6) δ 11.69 (s, 1H), 8.60 (s, 1H), 8.15 (dd, J = 5.0, 2.0 Hz, 1H), 7.62-7.53 (m,1H), 7.45-7.37 (m, 2H), 7.30 (d, J = 8.4 Hz, 2H), 7.15 (m, 4H), 7.14-7.09 (m, 1H), 5.42-5.15 (s, 2H), 4.62 (s, 1H), 3.88 (m, 2H), 3.01 (s, 3H), 2.69 (m, 1H), 0.98 (m, 3H).

In addition, the present inventors synthesized the exemplary compounds as in Table 3 below by using the aforementioned methods.

**Table 3**

| **Ex.** | **Structure** | **ESI-MS: *m*/*z*** | **Ex.** | **Structure** | **ESI-MS: *m*/*z*** |
|---|---|---|---|---|---|
| | | **(M+H )⁺** | | | **(M+H )⁺** |
| 25 | 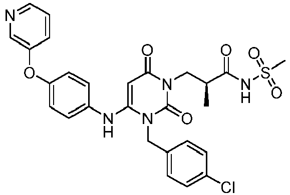 | 584.1 | 26 | 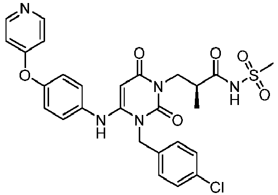 | 584.1 |
| 27 | 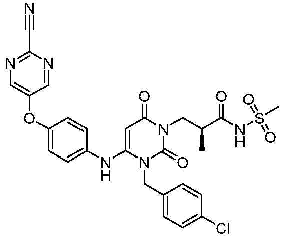 | 610.1 | 28 | 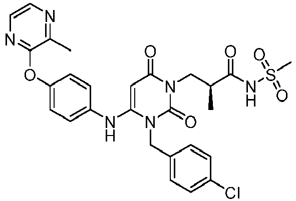 | 599.2 |
| 29 | 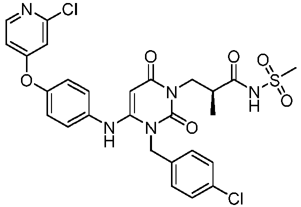 | 618.1 | 30 | 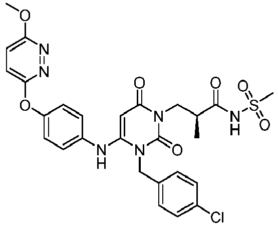 | 615.2 |
| 31 | 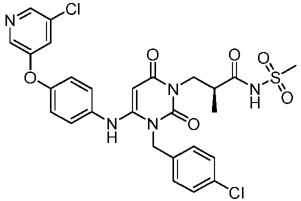 | 618.1 | 32 | 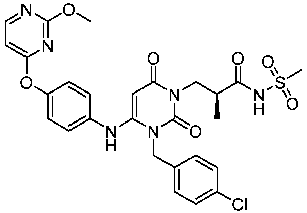 | 615.2 |
| 33 | 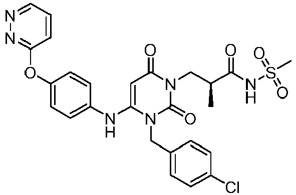 | 585.1 | 34 | 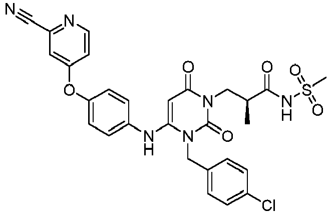 | 609.1 |
| 35 | 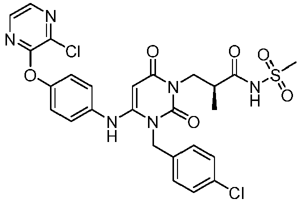 | 619.1 | 36 | 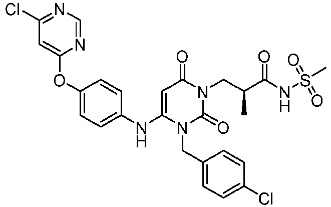 | 619.1 |
| 37 | 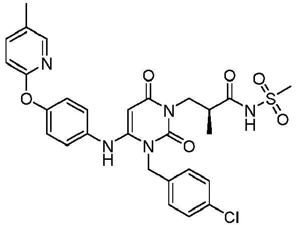 | 598.2 | 38 | 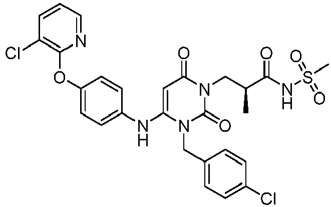 | 618.1 |
| 39 | 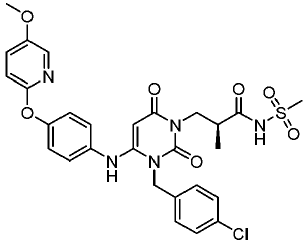 | 614.2 | 40 | 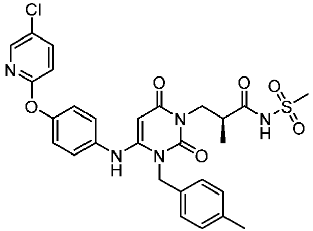 | 598.2 |
| 41 | 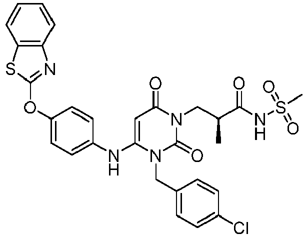 | 640.1 | 42 | 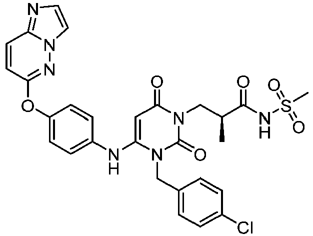 | 624.2 |
| 43 | 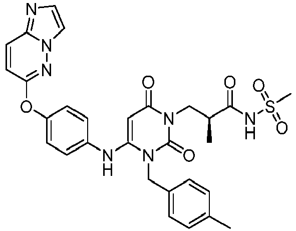 | 604.2 | 44 | 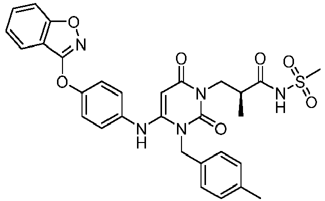 | 604.2 |
| 45 | 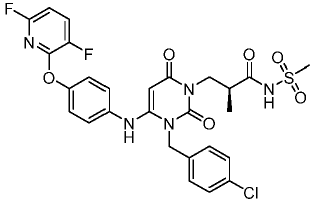 | 620.1 | 46 | 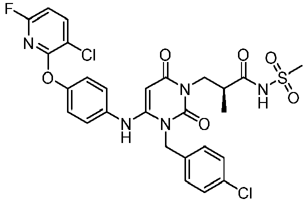 | 636.1 |
| 47 | 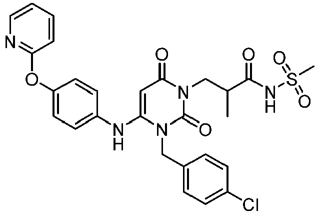 | 584.1 | 48 | 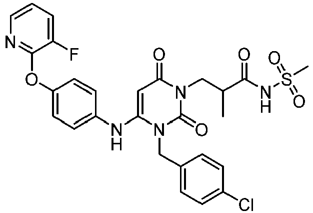 | 602.1 |
| 49 | 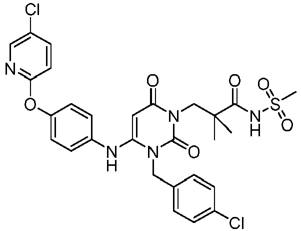 | 632.1 | 50 | 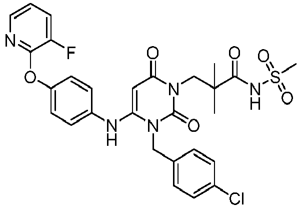 | 616.2 |
| 51 | 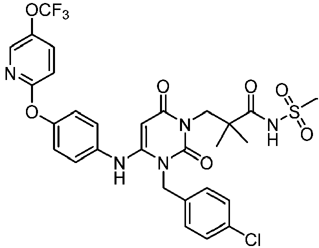 | 682.2 | 52 | 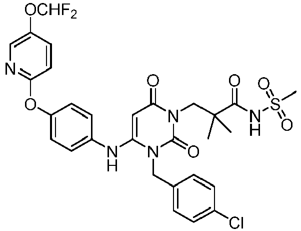 | 664.2 |
| 53 | 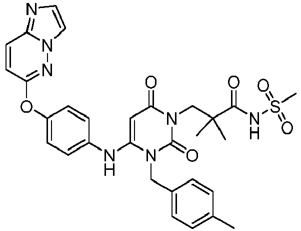 | 618.2 | 54 | 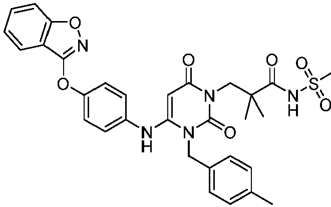 | 618.2 |
| 55 | 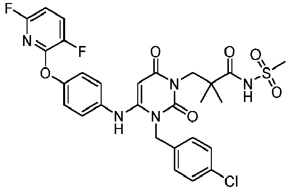 | 634.1 | 56 | 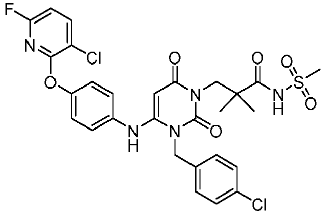 | 650.1 |
| 57 | 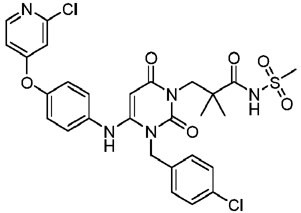 | 632.1 | 58 | 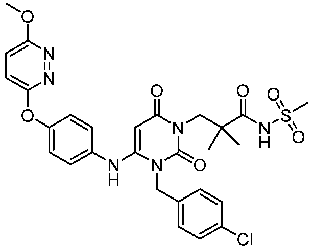 | 629.2 |

**Comparative Example 1:** Preparation of (S)-3-(3-(4-chlorobenzyl)-4-(4-(3-fluoropyridin-2-oxyl)phenyl)amino)-2,6-dioxo-3,6-dihydropyrimidin-1(2H)yl)-2-methylpropionic acid (Compound of Comparative Example 1)

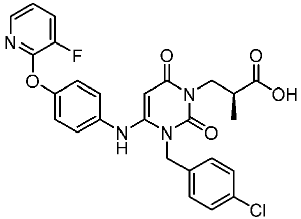

The preparation method of this comparative example was conducted under the same conditions as those in Example 2, with the exception that 2-fluoropyrazine in Step I was replaced with an equimolar of 2,3-difluoropyridine, to obtain a white solid-like compound of Comparative Example 1, yield: 81.6%, purity: 99.21%.

ESI-MS: *m*/*z* = 525.1 (M+H) ⁺.

¹H NMR (400 MHz, DMSO-d6) δ 12.09 (s, 1H), 8.65 (s, 1H), 8.03 (q, J = 8.2 Hz, 1H), 7.44 (d, J = 8.2 Hz, 2H), 7.32 (d, J = 8.2 Hz, 2H), 7.22 (brs, 4H), 6.95 (dd, J = 8.0, 1.7 Hz, 1H), 6.90 (dd, J = 7.9, 2.5 Hz, 1H), 5.31 (s, 2H), 4.66 (s, 1H), 4.08-3.80 (m, 2H), 2.77 (m, 1H), 0.99 (m, 3H).

### Test Example 1: Mouse cough test

### 1. Test material

### 1.1 Basic information of the test materials

Compounds of Example 1-24 (synthesized in the inventor's laboratory), positive control drug (gefapixant, batch No: 01030-210326-2-1, purchased from Holder Pharmaceuticals), the compound of Comparative Example 1 (synthesized in the inventor's laboratory).

### 1.2 Test reagents

### Physiological saline, ammonia.

### 2. Experimental animals

Healthy adult KM mice, half of them are male and half of them are female, 6 per group, weighing about 28-30g.

### 3. Test method

### 3.1 Dosage design and amount of test material used

Most of the animal coughing models currently reported in the literatures use mechanical, chemical and electrical stimulations to stimulate the nerves and receptors of the animals to trigger coughing. According to the characteristics of the candidate compounds and the existing similar target compounds for reference, the method of concentrated ammonia induction was initially chosen to establish the cough modeling test in mice.

### 3.2 Preparation of test materials

Preparation of 50% ammonia solution: 2.5 ml of ammonia was dissolved in 5 ml of a 0.9% sodium chloride solution for injection and mixed evenly.

Preparation of a solution of the compound of Comparative Example 1: 9mg of the compound of Comparative Example 1 was weighed and dissolved in 3ml of a 0.5% CMC-Na solution, and mixed evenly to prepare a 3mg/ml solution.

Preparation of a solution of the example compounds: 9 mg of the example compound was weighed and dissolved in 3ml of a 0.5% CMC-Na solution, and mixed evenly to prepare a 3mg/ml solution.

### 3.3 Experimental procedure

Grouping: a positive control group, a Comparative Example 1 group, Example 1 group- Example 24 group, and a model group were set up. Six KM mice were taken from each group and administered by gavage; here, the Comparative Example 1 group was given the compound of Comparative Example 1, the positive control group was given the positive control drug (gefapixant, commercially purchased), the Example groups were given the compounds prepared in the corresponding Examples, with each group given one of three dosages: 30 mg/kg, 10 ml/kg, and 3 mg/kg; and the model group was given the same volume of 0.5% CMC-Na solution.

30 min, 60 min or 120 min after administration, the mice were placed in a 500 ml beaker with a cotton ball (weight 100±5mg) containing 0.3 ml of 50% ammonia. The number of typical coughing of the mice (typical coughing action: contraction of the abdominal muscles or chest contraction, with mouth wide opening and a coughing sound) was observed in 3 min.

### 4. Results and Discussion

### 4.1 Judgment criteria of the results

(1) Judgment criteria of coughing: coughing was manifest in contraction of abdominal muscles or chest contraction, with mouth wide opening and a coughing sound.
(2) The number of coughs (times) within 3 min was recorded with a stopwatch, and the data were statistically analyzed by software, with the data in each group shown in mean ± standard deviation. A one-way analysis of variance among the groups was conducted, and P<0.05 was considered a difference having a statistical significance.

### 4.2 Discussion of the results

### 4.2.1 The number of coughs in mice 30min after administration of the example compounds at 30mg/kg

**Table 4**

| **Group** | **Number of Coughs (time)** | **Group** | **Number of Coughs (time)** |
|---|---|---|---|
| Model group | 50.50 | Example 12 group (Compound 12) | 15.67** |
| Positive control group | 20.33** | Example 13 group (Compound 13) | 25.60* |
| Comparative Example 1 group | 25.61* | Example 14 group (Compound 14) | 32.17 |
| Example 1 group (Compound 1) | 15.33** | Example 15 group (Compound 15) | 18.64** |
| Example 2 group (Compound 2) | 27.67* | Example 16 group (Compound 16) | 19.34** |
| Example 3 group (Compound 3) | 16.21** | Example 17 group (Compound 17) | 21.10** |
| Example 4 group (Compound 4) | 15.61** | Example 18 group (Compound 18) | 20.57** |
| Example 5 group (Compound 5) | 33.67 | Example 19 group (Compound 19) | 17.62** |
| Example 6 group (Compound 6) | 17.81** | Example 20 group (Compound 20) | 15.17** |
| Example 7 group (Compound 7) | 15.20** | Example 21 group (Compound 21) | 18.83** |
| Example 8 group (Compound 8) | 25.00* | Example 22 group (Compound 22) | 35.61 |
| Example 9 group (Compound 9) | 13.62** | Example 23 group (Compound 23) | 34.67 |
| Example 10 group (Compound 10) | 17.20** | Example 24 group (Compound 24) | 17.07** |
| Example 11 group (Compound 11) | 31.00 | / | / |

| | | | |
|---|---|---|---|
| Note: compared with the model group: **P<0.01, *P<0.05. | | | |

As seen from the above table, the number of coughs in mice induced by ammonia 30 min after the administration was significantly reduced in the positive control group compared with the model group, with a statistical difference (P<0.01), indicating that the modeling was successful. The number of coughs was significantly reduced in several example compound groups compared with the model group, with a statistical difference (P<0.01, P<0.05); and the cough suppressing effect of the inventive compounds of Examples 1, 3, 4, 6, 7, 9, 10, 12, 15, 19, 20 and 24 was stronger than that of the positive compound.

### 4.2.2 Number of coughs in mice 60min after administration of different doses

**Table 5**

| **Group** | **Number of Coughs (time)** | | |
|---|---|---|---|
| | **3mg/kg** | **10mg/kg** | **30mg/kg** |
| Model group | 49.17 | 50.03 | 48.90 |
| Positive control group | \ | 38.83 | 23.83** |
| Example 3 group (Compound 3) | 21.14 ** | 19.83**^{△} | 16.02** |
| Example 7 group (Compound 7) | 23.34** | 20.83**^{△} | 15.00** |
| Example 8 group (Compound 8) | \ | \ | 27.33* |
| Example 9 group (Compound 9) | 25.37 ** | 19.54**^{△} | 16.33 ** |
| Example 10 group (Compound 10) | \ | \ | 40.17 |
| Example 12 group (Compound 12) | 33.17 | 21.17**^{△} | 17.17** |
| Example 15 group (Compound 15) | 24.83 ** | 20.83**^{△} | 16.33** |
| Example 16 group (Compound 16) | 23.81 ** | 15.13**^{△} | 16.11 ** |
| Example 24 group (Compound 24) | 25.45 ** | 24.14**^{△} | 21.33** |

| | | | |
|---|---|---|---|
| Note: compared with the model group: **P<0.01, *P<0.05; compared with the positive control group: ^{△}P<0.05. | | | |

As shown in Table 5, with ammonia-induced coughing in mice 60 min after the administration, the number of coughs was significantly reduced in the 30 mg/kg positive control group compared with the model group, with a statistical difference (P<0.01); whereas the reduction in the number of coughs in the 10 mg/kg positive control group was not statistically significant when compared with the model group, suggesting that the 10 mg/kg positive control drug did not have a significant effect in reducing coughs, and that the 10 mg/kg positive control drug did not have any significant cough-reducing effect. However, at this dose (10 mg/kg), the number of coughs in groups having the compounds of Examples 3, 7, 9, 12, 15, 16, and 24 were statistically significantly reduced in t compared with the positive control group (^{△}P<0.05), and also were statistically significantly reduced in compared with the model group (**P<0.01), suggesting that these example compounds had improved cough suppressing efficacy than the positive control drug; the compounds of Examples 3, 7, 9, 15, 16, and 24 even showed statistically significant (P<0.01) reduction in the number of coughs when the dosage was lowered to 3 mg/kg. In addition, in the Example 10 group, the cough suppressing effect was obvious at 30 min, but no cough suppressing effect at 60 min, indicating that the compound in the Example 10 group had a short duration of action.

### 4.2.3 Number of coughs 60 and 120min after administration of 30mg/kg of test samples

**Table 6**

| **Group** | **Number of Coughs (time)** | |
|---|---|---|
| | **60min** | **120min** |
| Model group | 48.90 | 51.05 |
| Comparative Example 1 group | 24.12** | 44.17 |
| Example 3 group (Compound 3) | 16.02** | 15.13**^{△} |
| Example 7 group (Compound 7) | 15.00** | 38.13 |
| Example 9 group (Compound 9) | 16.33 ** | 23.83**^{△} |
| Example 12 group (Compound 12) | 17.17** | 20.17*^{△} |
| Example 15 group (Compound 15) | 16.33** | 23.83**^{△} |
| Example 16 group (Compound 16) | 16.11** | 18.13**^{△} |
| Example 24 group (Compound 24) | 21.33** | 25.14**^{△} |

| | | |
|---|---|---|
| Note: compared with the model group: **P<0.01, *P<0.05; compared with the Comparative Example 1 group: ^{△}P<0.05. | | |

As shown in Table 6, with administration of the test drug at 30 mg/kg, the number of coughs of mice in the Comparative Example 1 group compared with the model group was significantly reduced 60 min after the administration, which was statistically different (P<0.01); and the reduction in the number of coughs in the Comparative Example 1 group was not statistically significant compared with the model group 120 min after the administration, indicating that the compound in the Comparative Example 1 group did not have a significant cough suppressing effect at 120 min. As for the compounds of Examples 3, 9, 12, 15, 16 and 24, the number of coughs was significantly reduced both 60 min and 120 min after the administration, which were statistically different from that of the model group (P<0.01), and statistically different from that of the compound of Comparative Example 1 at 120 min (P<0.05), suggesting that the duration of the cough suppression action of the compounds of Example 3, 9, 12, 15, 16 and 24 was significantly prolonged compared with that of the compound of Comparative Example 1.

### Test Example 2: In vitro bioactivity evaluation

The reagents, consumables and instruments used in this test example were commercially available.

### 1. Cell lines

The HEK 293 cell line stably transfected with human P2X3 receptor was used.

### 2. Cell culture

Cell culture medium: DMEM high glucose; 10% FBS; 1% PenStrep.

### 3. Cell culture protocol

### a) Cell recovery

1) The cell cryopreservation tube was immersed in a water bath at 37°C and shaken continuously to allow it to thaw as quickly as possible.
2) The cells were gently blown up and down with a 1 mL pipette gun to make them suspend, and added dropwise to a 15 mL centrifuge tube containing 10 mL of fresh prewarmed growth medium, and centrifuged at 1000 rpm/min for 5 minutes.
3) The supernatant was discarded, and the cells were resuspended in 5 mL of fresh growth medium. The cell suspension was transferred to a petri dish and placed in a 5% CO₂ incubator at 37°C for static incubation.
4) After 24 hours, the medium was slowly removed (care was taken not to disrupt the cell monolayer), and incubation was carried out with a fresh growth medium.

### b) Passaging

Cell lines are usually passaged twice a week at a dilution of 1:3 to 1:4 (a 1:3 passaging ratio was more commonly used), and the cells after passaging needed 2-3 days before they grew to 85% confluence.
1) After the cells reached >85% saturation in a 10 cm dish, the cells were digested with a 0.25% Trypsin-EDTA solution for about 1 min, and the cells are aspirated out of the dish.
2) The cells were transferred to a dish containing a complete growth medium according to the dilution ratio. Note: to maintain a logarithmic growth of cells, the cells should be maintained in a monolayer culture.
3) Depending on the cell multiplication time of the cell line (HEK293-P2X3: 24 hours), the cells were passaged using a 0.25% trypsin solution.

### c) Cryopreservation of cells

1) The petri dish was removed from the incubator and placed on an ultra-clean bench, digested with a 0.25% Trypsin-EDTA solution for about 1 min, and the cells were collected and counted, and then centrifuged at 1000 rpm/min for 5 min.
2) The supernatant was spirated out, and the cells were resuspended in a cryopreservation solution (90% FBS and 10% DMSO) at a density of 2×10⁶ cells/mL, with 1 mL of cell suspension added to each cryopreservation tube.
3) The cell cryopreservation tube was placed in a cryopreservation box and transferred to -80°C overnight.
4) The cell cryopreservation tube was placed in a liquid nitrogen tank (-196°C).

### 4. Experimental procedure

### Step 1: Preparation of cell plates

1) After the cells grew to 80% confluence in a 15 cm Petri dish, the supernatant was removed, the cells were washed by adding 5 mL of DPBS and aspirated, and then 2.5 mL of a 0.25% Trypsin-EDTA solution was added to the Petri dish. The Petri dish was placed in an incubator for 1-3 minutes or until the cells were digested, 3 mL of a complete medium was then added to terminate the digestion, and the cell density was determined by a cell counter.
2) After centrifugation at 1000 rpm/min for 5 min, the cells were resuspended in a growth medium and the volume of the suspension was adjusted to reach a cell density of 4×10⁵ cells/mL (1×10⁴ cells/25 µL).
3) 10 µL of 5×Matrigel was added into a black microplate, and the microplate was placed in an incubator for 15 min. The microplate was removed and invertedly centrifuged at 300 g/min for 30 s to remove the 5×Matrigel. Then, the prepared cell suspension was added into a 384 black microplate, with 25 µL per well.
4) The microplate was placed into an incubator with 5% CO₂ at 37°C overnight, and by the next day the cells grew to confluence.

### Step 2: Compound preparation

### Antagonist mode

1) Masterbatch concentration of the test compounds: 20 mM.
2) The compound was diluted in a 384-LDV plate with a 12-point dilution using Bravo, with an initial concentration of the compound at 10 µM and dilution in 3-folds.
3) HPE (High Potency Effect Control): single dose of the positive control compound; FAC (Final Active Concentration): 40 µM; ZPE (Zero Effect Control): 100% DMSO.
4) The compounds on the 384-LDV plate, the HPE and the ZPE were transferred by ECHO to a 384-well plate (PE 6008590) as a compound plate.
5) The compound plate was stored at -20°C.

### Step 3: Screening test

1) Cell plates grown to confluence were removed from the incubator.
2) Preparation of assaying buffer: 30 mL of a buffer containing 0.3 mL of 250 mM probenecid, 0.6 mL of 1M HEPES and 29.1 mL of HBSS (the actual amount of the assaying buffer depended on the number of cell plates).
3) Preparation of C6 dye: with a C6 dye stock solution at 10×, the C6 dye was diluted to 1× with the buffer.
4) Inverted centrifugation was conducted using Bluewasher in the gentle spin mode to remove the culture medium.
5) The C6 dye was added to the cell plate with a pipette pipette gun at 20 µL/well.
6) The cell plate was centrifuged at 300 rpm/min for 30 s and incubated in an incubator for 1.5 h.
7) 20 µL of the experimental buffer was added to each well of the pre-prepared compound plate using a Dragonfly autosampler, and 10 µL of the compound was transferred to the cell plate using Bravo according to the layout of the experimental plate, with the final assay concentration of the compound at the highest dose FAC: 10 µM, 3-fold dilution, 12 concentration points.
8) The cell plate was centrifuged at 300 rpm/min for 30 s and incubated in an incubator for 30 min.
9) 25 µL 4×BZATP (final concentration 3.5 µM) agonist was preprared on an agonist plate (PE 6008590) to act on P2X3 cells.
10) The cell plate, a FLIPR gun head and the agonist plate were placed at room temperature for 15 min.
11) 10 µL of the BZATP agonist was transferred to the cell plate by FLIPR and the number was read.

### Step 4: Experimental results and analysis

The IC50 inhibition of the Example compounds to the P2X3 receptor is shown in the table below, where A indicates less than 10 nM, B indicates 10.1-50 nM, and C indicates 50.1-100 nM.

**Table 7**

| **Test sample** | **P2X3 IC₅₀ (nM)** | **Test sample** | **P2X3 IC₅₀ (nM)** |
|---|---|---|---|
| Positive control drug | C | Compound 11 | B |
| Comparative Example 1 | B | Compound 12 | A |
| Compound 1 | A | Compound 13 | B |
| Compound 3 | B | Compound 14 | B |
| Compound 4 | A | Compound 15 | B |
| Compound 6 | B | Compound 16 | A |
| Compound 7 | A | Compound 20 | B |
| Compound 8 | B | Compound 22 | C |
| Compound 9 | B | Compound 23 | C |
| Compound 10 | A | Compound 24 | A |

The results show that the compounds of the present invention have improved inhibitory effect to the P2X3 receptor in vitro than the positive control drug and the compound of Comparative Example 1.

### Test Example 3: Taste disorder test

### 1. Test material

### 1.1 Basic information of test materials

Compounds of Examples 1, 9, 12, 15, 16, 24 (synthesized in the laboratory of the present inventor), the positive control drug (gefapixant, batch No: 01030-210326-2-1, purchased from Holder Pharmaceuticals).

### 1.2 Test reagents

0.9% sodium chloride solution for injection, quinine hydrochloride (Quinie, batch No: C12476271)

### 2. Experimental animals

Healthy adult SD rats, all males, weighing about 280-300g.

### 3. Test method

### 3.1 Preparation method of the test material

Preparation of 0.3mM quinine solution: 119.20mg of quinine hydrochloride was weighed and dissolved in 1000ml of tap water, and mixed evenly.

Preparation of the test sample solution: 40mg of a test sample was dissolved in DMSO and a solution of the solubilizer HS-15 was added, mixed evenly, and 16ml of saline was added to formulate a 2.5mg/ml solution.

### 3.2 Experimental procedure

Animal and grouping: male SD rats weighed about 160-180g each, with 10 of a similar average weight in each group, were housed in seperate cages.

Drinking habit training: each group was given normal drinking water for 30 minutes at 8:30 am and 16:30 pm every day, and no access of water for the rest of the day for 5 days, and two bottles of water were changed every day at the left and right positions.

Administration: with no access to water the night before the experiment, in the following morning the test group was given 4 mL/kg (10 mg/kg) of the test sample by tail intravenous injection, and the model group was given 4 mL/kg (10 mg/kg) of 0.5% HS-15 by intravenous injection.

### 4. Results and discussion

### 4.1 Judgment criteria of results

Measurement of water intake: after the injection, the animals were put back to their previous cages, and the measurement time of each group was within the Tmax interval of each drug (the measurement time was 0min-15min after the administration), and one bottle of normal drinking water and one bottle of drinking water containing 0.3mM quinine hydrochloride (Quinie) were both placed in each cage, with the left and right positions of the two bottles of water being the same in all the animal cages. The animals were allowed to drink freely for 15 min, and the amount of water consumed from the two bottles was measured to the nearest 0.1 ml.

(2) Statistical analysis of the data: the drinking volume of the quinine bitter water and tap water were recorded separately, as well as the percentage of quinine water to the tap water, and the significance of the difference between the groups was determined by ANOVA.

### 4.2 Discussion of results

**Table 8**

| **Test sample** | **Quinine / tap water (%)** |
|---|---|
| Vehicle group | 38.16% |
| Positive control group | 79.01%** |
| Example 1 group (Compound 1) | 41.48%^{△} |
| Example 9 group (Compound 9) | 39.84%^{△} |
| Example 12 group (Compound 12) | 37.18%^{△} |
| Example 15 group (Compound 15) | 40.22%^{△} |
| Example 16 group (Compound 16) | 42.53%^{△} |
| Example 24 group (Compound 24) | 39.61%^{△} |

| | |
|---|---|
| Note: compared with the vehicle group: **P<0.01; compared with the positive control group: ^{△}P<0.01. | |

As seen from Table 8, the ratio of quinine/tap water consumed by mice in the positive control group was statistically different from that in the vehicle group (P<0.01), indicating that the compound gefapixant in the positive control group had a significant effect on the taste sense of mice. In contrast, in the groups of Example compounds 1, 9, 12, 15, 16 and 24, the ratio of quinine/tap water consumed by the mice was not statistically different from that in the vehicle group, indicating that the compounds of the present invention had little or no effect on the taste sense of the mice when administered intravenously at 10 mg/kg, which was a statistically significant difference from that of the positive control group.

The above examples are only preferred embodiments of the present invention, and should not be construed as limitation to the protection scope of the present invention. Any non-substantive alteration or modification made in within the principal idea and spirit of the present invention, which solve a technical problem consistent with that of the present invention, should be encompassed within the protection scope of the present invention.

## Claims

1. A compound having a structure represented by Formula I, or a salt, a solvate, an isomer, a metabolite, a nitrogen oxide, or a prodrug thereof: 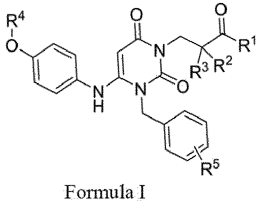 wherein,
R¹ is selected from hydroxyl, a substituted or unsubstituted alkoxy, a substituted or unsubstituted sulfonamide group, a substituted or unsubstituted heterocyclic epoxy group, and a substituted or unsubstituted aryloxy; R² and R³ are independently selected from hydrogen, deuterium, and a substituted or unsubstituted C₁₋₁₂ alkyl; or R² and R³ connect to each other to form a substituted or unsubstituted 3 to 15-membered cycloalkyl; R⁴ is selected from a substituted or unsubstituted heterocyclic group, and a substituted or unsubstituted aryl; R⁵ is selected from hydrogen, deuterium, a substituted or unsubstituted alkyl, a substituted or unsubstituted alkoxy, a substituted or unsubstituted alkylthio, a substituted or unsubstituted amino, and halogen; and the compound of Formula I does not include the following compounds:

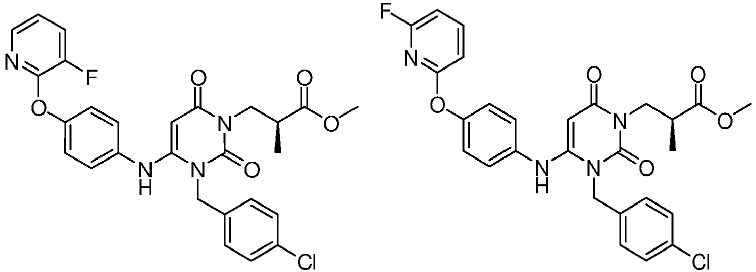

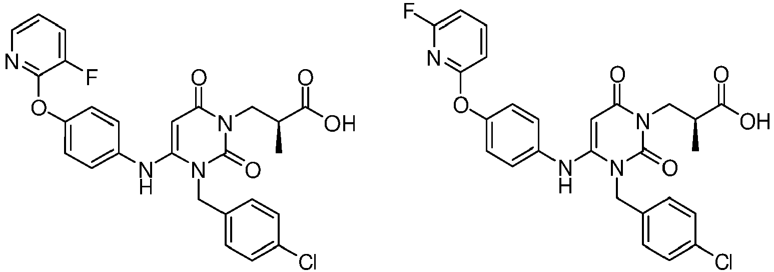

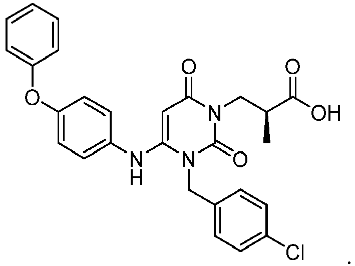

2. The compound, or a salt, a solvate, an isomer, a metabolite, a nitrogen oxide, or a prodrug thereof according to claim 1, wherein R⁴ is selected from a substituted or unsubstituted phenyl, a substituted or unsubstituted pyridyl, a substituted or unsubstituted pyrimidinyl, a substituted or unsubstituted pyrazinyl, a substituted or unsubstituted pyridazinyl, a substituted or unsubstituted benzothiazolyl, a substituted or unsubstituted benzisoxazolyl, or a substituted or unsubstituted imidazopyridazinyl.

3. The compound, or a salt, a solvate, an isomer, a metabolite, a nitrogen oxide, or a prodrug thereof according to claim 1 or 2, wherein R¹ is selected from hydroxyl, a substituted or unsubstituted alkoxy, and a substituted or unsubstituted sulfonamide group; and/or R⁴ is selected from a substituted or unsubstituted phenyl, a substituted or unsubstituted pyridyl, a substituted or unsubstituted pyrimidinyl, a substituted or unsubstituted pyrazinyl, a substituted or unsubstituted pyridazinyl, a substituted or unsubstituted benzothiazolyl, a substituted or unsubstituted benzisoxazolyl, and a substituted or unsubstituted imidazopyridazinyl, wherein the substituent of R⁴ is selected from 1 to 5 of hydrogen, deuterium, amino, cyano, halogen, a substituted or unsubstituted alkyl, and a substituted or unsubstituted alkoxy; and/or **R⁵** is selected from 1 to 5 hydrogen, deuterium, amino, halogen, and a substituted or unsubstituted alkyl.

4. The compound, or a salt, a solvate, an isomer, a metabolite, a nitrogen oxide, or a prodrug thereof according to claim 1 or 2, wherein R² and R³ are independently selected from hydrogen, deuterium, substituted or unsubstituted methyl, substituted or unsubstituted ethyl, substituted or unsubstituted propyl, and substituted or unsubstituted cyclopropyl; preferably, when R² and R³ are independently selected from substituted methyl, substituted ethyl, substituted propyl, or substituted cyclopropyl, the substituent of R² and R³ are independently selected from one or more deuterium, halogen, hydroxyl, amino, methyl, ethyl, cyclopropyl, tert-butyl, methoxy, ethoxy, cyclopropoxy, tert-butoxy, methylthio, ethylthio, cyclopropylthio, tert-butylthio, amino, methylamino, ethylamino, cyclopropylamino, tert-butylamino, formamido, acetamido, cyclopropylamido, tert-butylamido, NO₂, CN, and CF₃; or R² and R³ connect to each other to form substituted or unsubstituted cyclopropyl, cyclohexyl, or cyclopentyl; wherein the substituent of the substituted cyclopropyl is selected from one or more deuterium, halogen, hydroxyl, amino, methyl, ethyl, cyclopropyl, tert-butyl, methoxy, ethoxy, cyclopropoxy, tert-butoxy, methylthio, ethylthio, cyclopropylthio, tert-butylthio, amino, methylamino, ethylamino, cyclopropylamino, tert-butylamino, formamido, acetamido, cyclopropylamido, tert-butylamido, NO₂, CN, and CF₃; and/or R¹ is selected from hydroxyl, a substituted or unsubstituted alkoxy, and a substituted or unsubstituted sulfonamide group; preferably, R¹ is selected from a substituted or unsubstituted alkoxy, and a substituted or unsubstituted sulfonamide group, and the substituent of R¹ is selected from one or more deuterium, halogen, hydroxyl, alkyl, cycloalkyl, alkenyl, alkynyl, aryl, alkoxy, cycloalkoxy, alkylamino, cycloalkylamino, alkylthio, cycloalkylthio, amido, NO₂, CN, and CF₃; and/or R⁴ is selected from a substituted or unsubstituted phenyl, a substituted or unsubstituted pyridyl, a substituted or unsubstituted pyrimidinyl, a substituted or unsubstituted pyrazinyl, a substituted or unsubstituted pyridazinyl, a substituted or unsubstituted benzothiazolyl, a substituted or unsubstituted benzisoxazolyl, and a substituted or unsubstituted imidazopyridazinyl; the substituent of R⁴ is selected from 1 to 5 deuterium, amino, cyano, methyl, methoxy, halogen, trifluoromethoxy, and difluoromethoxy; and/or R⁵ is selected from 1 to 5 hydrogen, deuterium, amino, halogen, trifluoromethyl, and difluoromethyl.

5. The compound, or a salt, a solvate, an isomer, a metabolite, a nitrogen oxide, or a prodrug thereof according to claim 1 or 2, wherein R² and R³ are independently selected from hydrogen, deuterium, methyl, ethyl, propyl, and cyclopropyl; or R² and R³ connect to each other to form cyclopropyl; and/or R¹ is selected from hydroxyl, a C₁-C₁₆ alkoxy, a C₁-C₁₆ alkylsulfonamide group, a C₁-C₆ alkylaminosulfonamide group, a C₁-C₆ cycloalkylaminosulfonamide group, and a substituted or unsubstituted benzenesulfonamide group; and/or R⁴ is selected from a substituted or unsubstituted phenyl, a substituted or unsubstituted pyridyl, a substituted or unsubstituted pyrimidinyl, a substituted or unsubstituted pyrazinyl, a substituted or unsubstituted pyridazinyl, a substituted or unsubstituted benzothiazolyl, a substituted or unsubstituted benzisoxazolyl, and a substituted or unsubstituted imidazopyridazinyl, wherein the substituent is selected from 1 to 5 deuterium, amino, cyano, methyl, methoxy, halogen, trifluoromethoxy, and difluoromethoxy; and/or R⁵ is selected from 1 to 5 hydrogen, deuterium, amino, methyl, halogen, trifluoromethyl, and a difluoromethyl.

6. The compound, or a salt, a solvate, an isomer, a metabolite, a nitrogen oxide, or a prodrug thereof according to claim 1 or 2, wherein R¹ is selected from hydroxyl, a C₁-C₁₆ alkoxy, a C₁-C₁₆ alkylsulfonamide group, a C₁-C₆ alkylaminosulfonamide group, and a C₁-C₆ cycloalkylaminosulfonamide group.

7. The compound, or a salt, a solvate, an isomer, a metabolite, a nitrogen oxide, or a prodrug thereof according to claim 1 or 2, wherein R⁴ is selected from 4-phenyl, 2-pyridinyl, 2-pyrimidinyl, 2-pyrazinyl, 3-pyridazinyl, 3-fluoropyridin-2-yl, (5-trifluoromethoxy)pyridin-2-yl, (5-difluoromethoxy)pyridin-2-yl, 5-chloro-pyridin-2-yl, 3-pyridinyl, 4-pyridinyl, 6-fluoro-pyridin-2-yl, 2-cyanopyrimidin-5-yl, 3-methylpyrazin-2-yl, 2-chloro-pyridin-4-yl, 6-methoxypyrimidin-3-yl, 5-chloropyridin-3-yl, 2-methoxypyrimidin-4-yl, 2-cyanopyridin-4-yl, 3-chloropyrazin-2-yl, 6-chloropyrimidin-4-yl, 5-methylpyridin-2-yl, 3-chloropyridin-2-yl, 5-methoxypyridin-2-yl, 5-chloropyridin-2-yl, benzo[D]thiazol-2-yl, imidazo[1,2-B]pyridazin-6-yl, benzo[D]isoxazol-3-yl, 3,6-difluoropyridin-2-yl, and 3-chloro-6-fluoropyridin-2-yl.

8. The compound, or a salt, a solvate, an isomer, a metabolite, a nitrogen oxide, or a prodrug thereof according to claim 1, which is selected from the following compound or a pharmaceutically acceptable salt thereof:

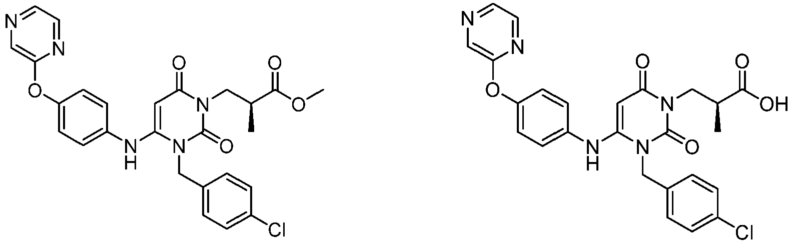

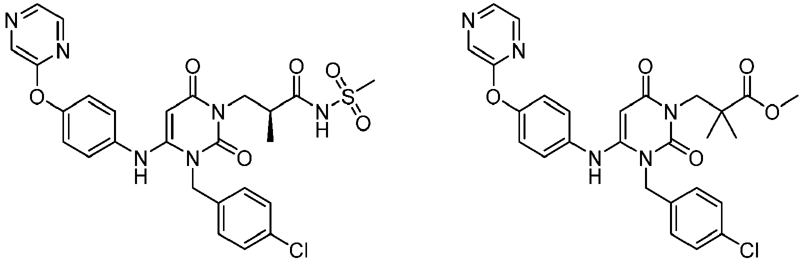

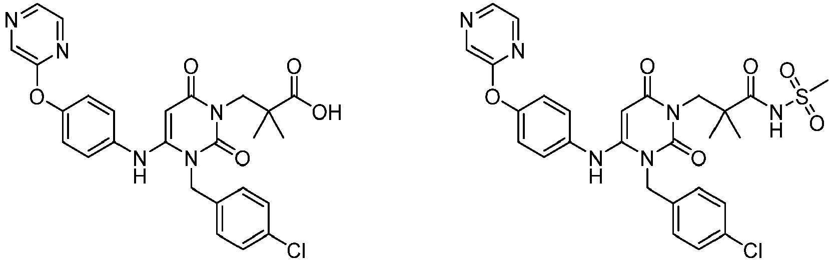

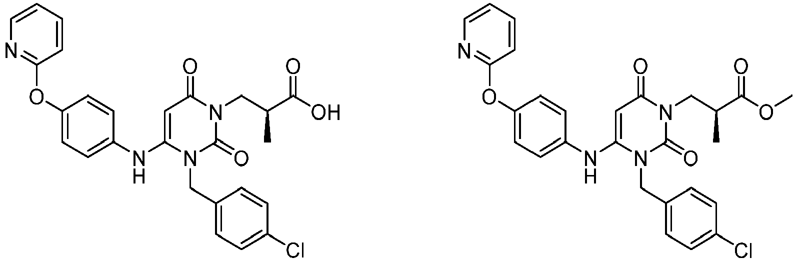

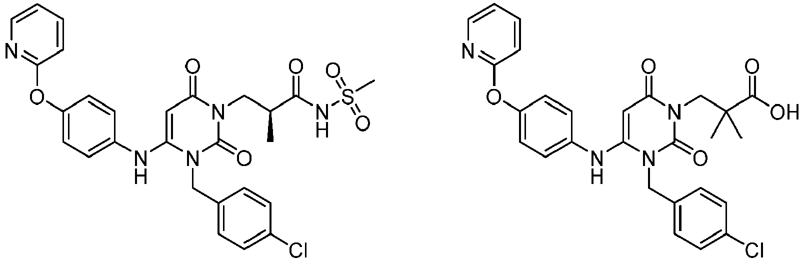

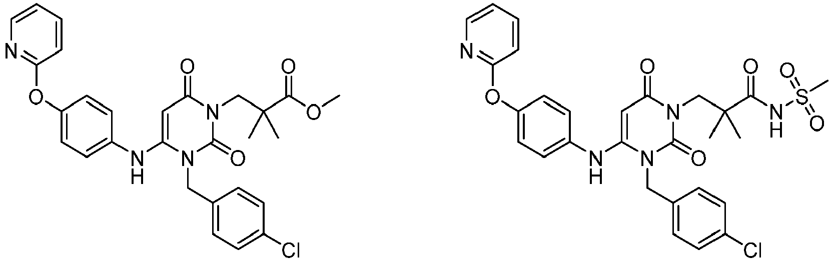

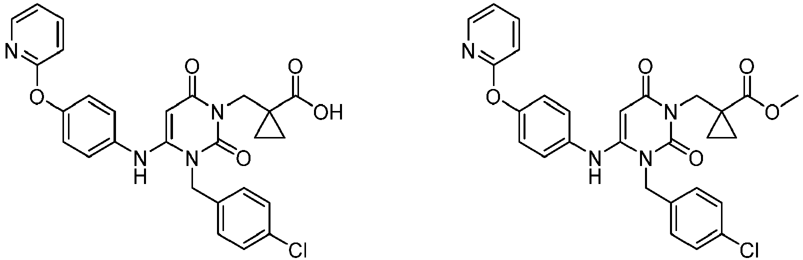

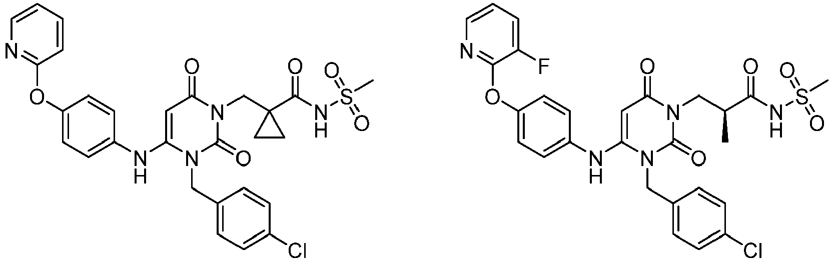

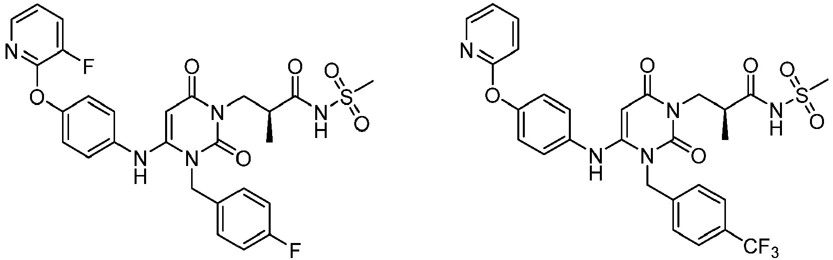

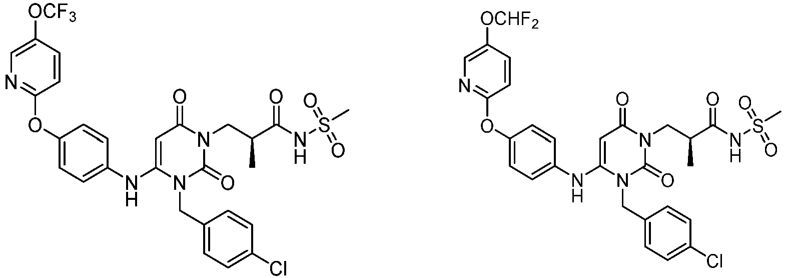

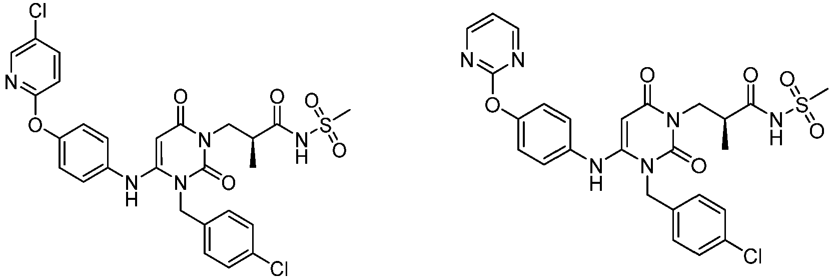

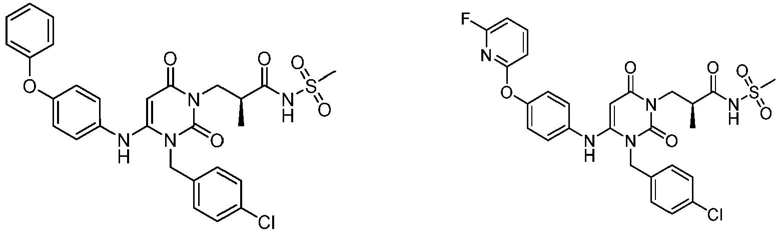

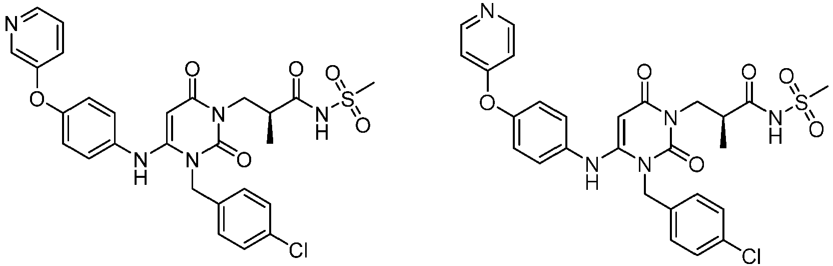

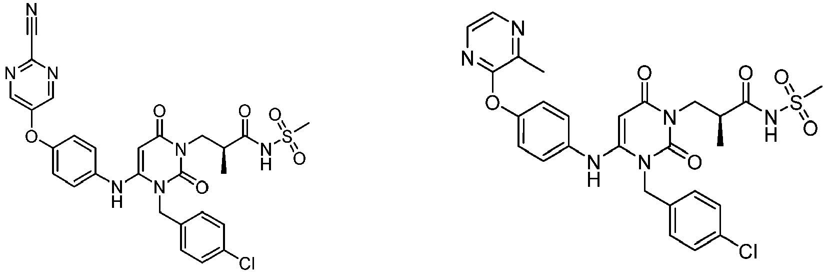

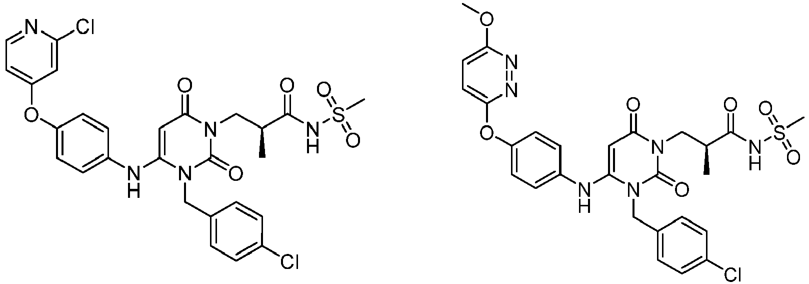

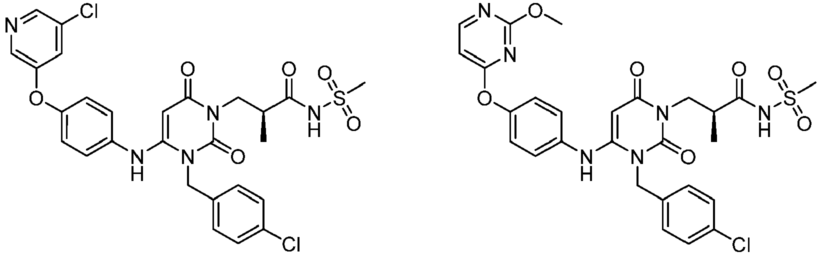

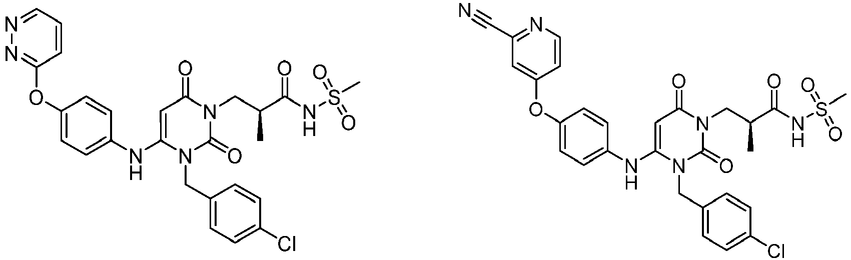

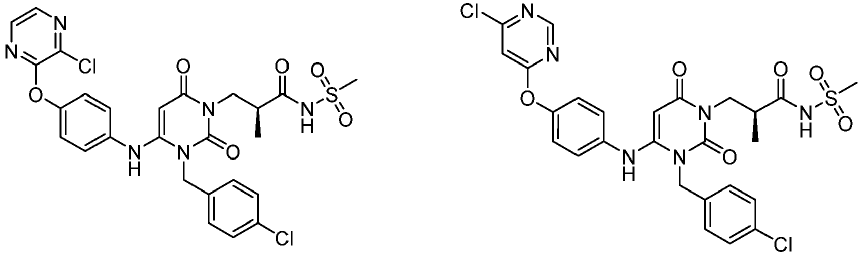

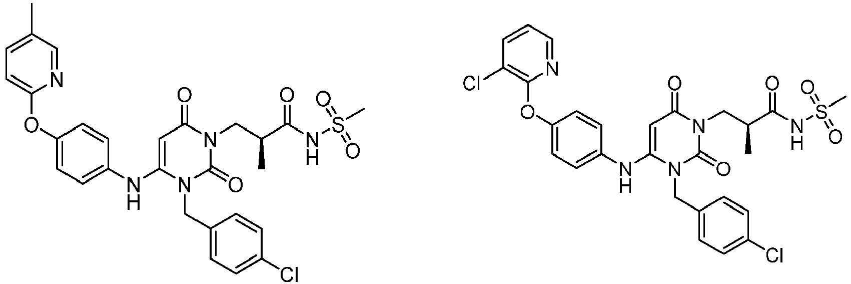

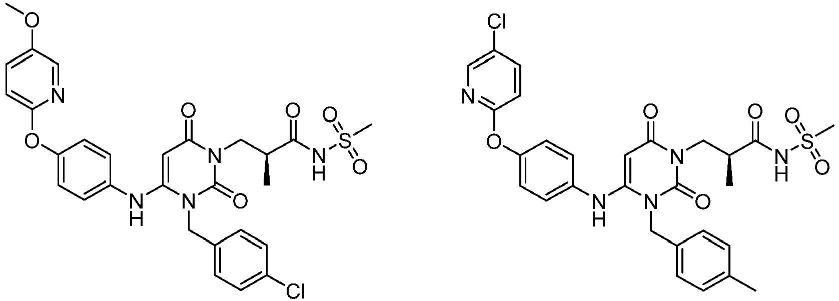

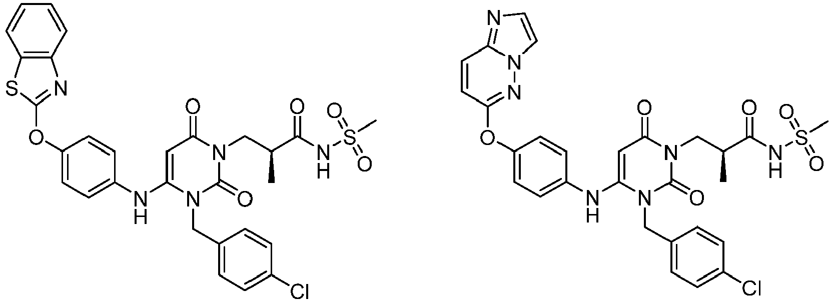

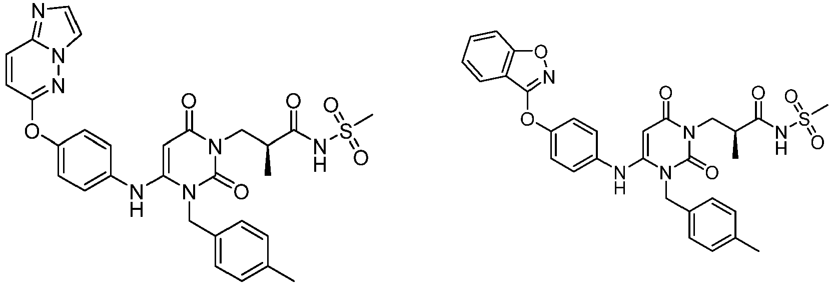

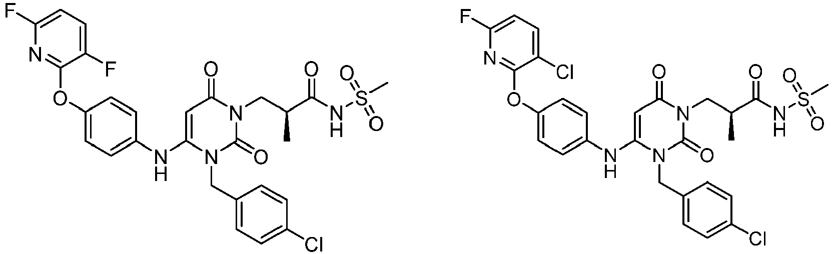

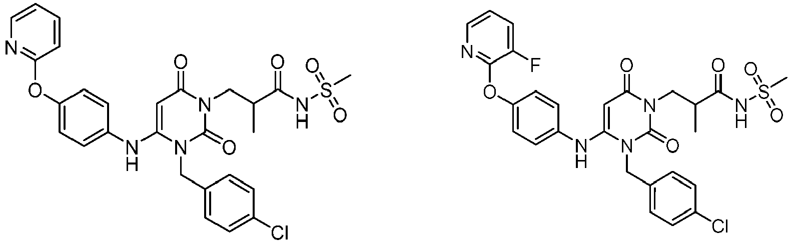

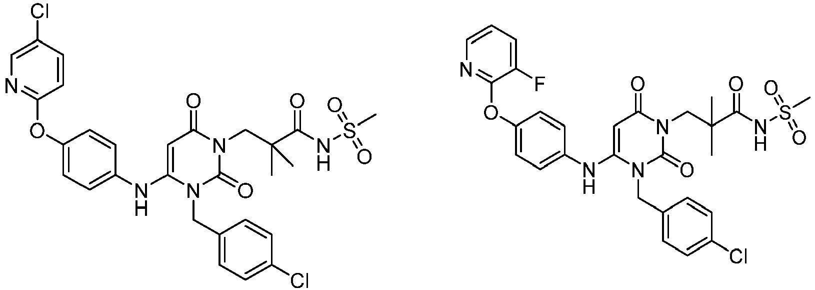

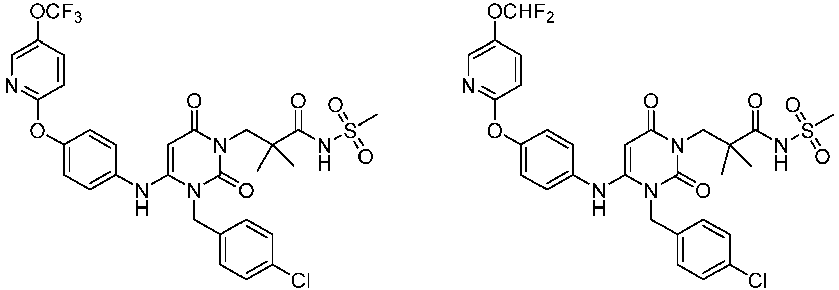

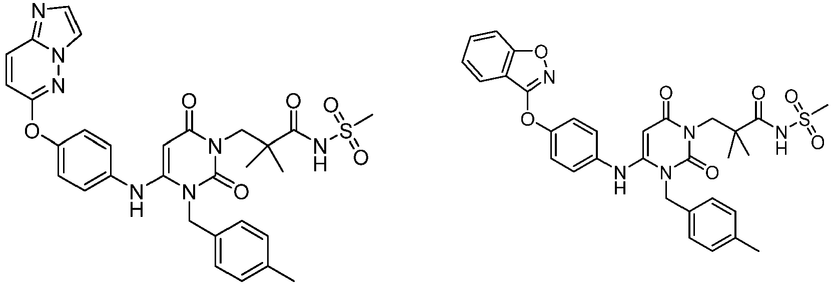

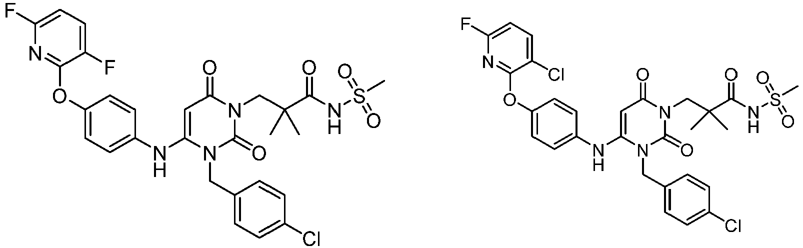

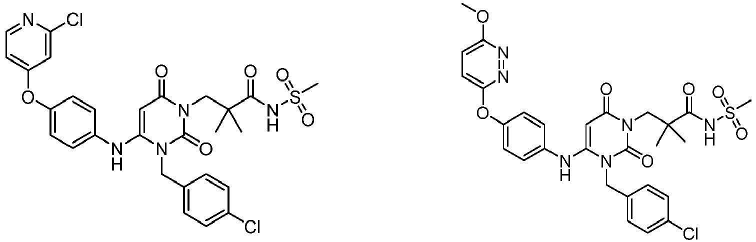

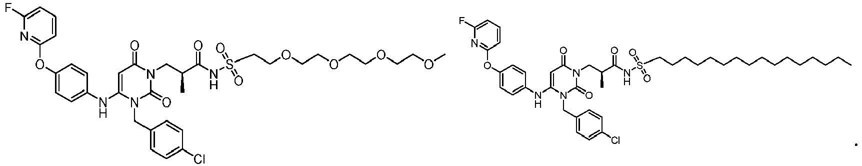

9. The compound, or a salt, a solvate, an isomer, a metabolite, a nitrogen oxide, or a prodrug thereof according to claim 1, wherein hydrogen in the compound can be substituted by one or more deuterium.

10. A method for preparing the compound or a salt, a solvate, an isomer, a metabolite, a nitrogen oxide, or a prodrug thereof according to any one of claims 1 to 9, wherein the method comprises the following steps:
when R¹ is methoxy:
Step I: subjecting compound **a** and compound **k** under an alkaline condition to a substitution reaction to produce compound **b;**
Step II: subjecting intermediate compounds **c** and **d** in the presence of an organic alkaline or an inorganic alkaline to a substitution reaction to produce **e;**
Step III: subjecting intermediate compounds **e** and **f** to a Mitsunobu reaction to produce an intermediate **g;**
Step IV: subjecting intermediate compounds **g** and **b** to a coupling reaction to produce an intermediate **h;** 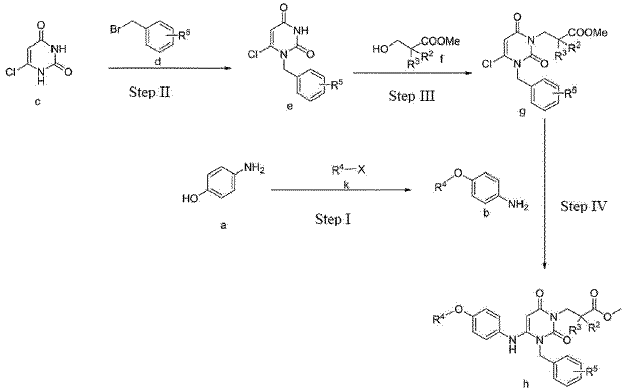
wherein R², R³, R⁴ and R⁵ are as defined in claim 1, and X is halogen;
or when R¹ is hydroxy, the method further comprises Step V: subjecting compound **h** under the catalysis of an inorganic alkaline or an acid to a hydrolysis reaction to produce compound **j;** 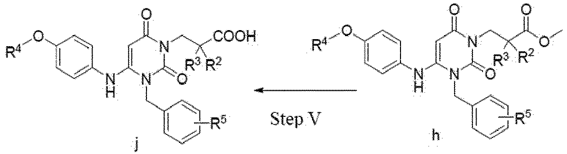
wherein R², R³, R⁴ and R⁵ are as defined in claim 1;
or when R¹ is selected from a substituted or unsubstituted alkoxy, a substituted or unsubstituted sulfonamide group, a substituted or unsubstituted heterocyclic epoxy group, and a substituted or unsubstituted aryloxy, and R¹ is not methoxy, the method further comprises Step VI: subjecting compound **j** and an alcohol-, sulfonamide group- or phenol-based compound having an R¹ group to a condensation or esterification reaction to produce a compound of formula I; 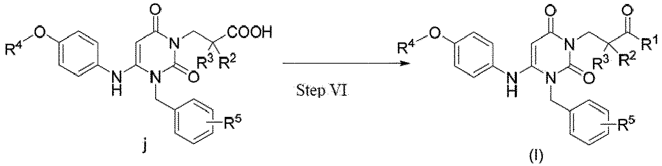
wherein R¹, R², R³, R⁴ and R⁵ are as defined in claim 1.

11. Use of the compound or a salt, a solvate, an isomer, a metabolite, a nitrogen oxide, or a prodrug thereof according to any one of claims 1 to 9 in the manufacture of a medicament for treating or preventing a disease associated with a P2X3 and/or P2X2/3 receptor, preferably for treating or preventing a respiratory disease, and more preferably for treating or preventing cough, asthma, pain, or sleep apnea.
